(19) Europäisches Patentamt
European Patent Office
Office européen des brevets

(11) Publication number: **0 400 835 A1**

(12) **EUROPEAN PATENT APPLICATION**

(21) Application number: 90305179.5

(22) Date of filing: 14.05.90

(51) Int. Cl.5: C07D 235/08, A61K 31/415, C07D 235/02, C07D 403/10, C07D 403/12, A61K 31/505, A61K 31/41, C07F 9/6506

(30) Priority: 15.05.89 US 351508
04.04.90 US 504441

(43) Date of publication of application:
05.12.90 Bulletin 90/49

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Chakravarty, Prasun K.
16 Churchill Road
Edison, NJ 08820(US)
Inventor: Patchett, Arthur A.
1090 Minisink Way
Westfield, NJ 07090(US)
Inventor: Camara, Valerie J.
80A Rivervale Court
Scotch Plains, NJ 07076(US)
Inventor: Walsh, Thomas F.
127 Marion Avenue
Westfield, NJ 07090(US)
Inventor: Greenlee, William J.
115 Herrick Avenue
Teaneck, NJ 07666(US)

(74) Representative: Hesketh, Alan, Dr. et al
European Patent Department Merck & Co., Inc. Terlings Park Eastwick Road
Harlow Essex, CM20 2QR(GB)

(54) **Substituted benzimidazoles as angiotensin II antagonists.**

(57) There are disclosed new substituted benzimidazole compounds and derivatives thereof which are useful as angiotensin II antagonists. These compounds have the general formula:

EP 0 400 835 A1

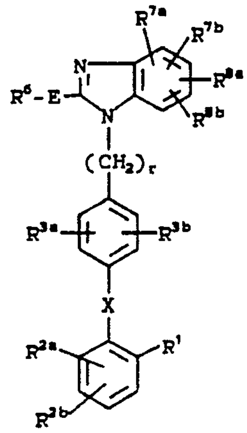

(I)

## SUBSTITUTED BENZIMIDAZOLES AS ANGIOTENSIN II ANTAGONISTS

### INTRODUCTION OF THE INVENTION

This invention relates to novel substituted benzimidazole compounds and dervatives thereof which are useful as angiotensin II antagonists in the treatment of elevated blood pressure and congestive heart failure. Thus, the substituted benzimidazole compounds of the invention are useful as antihypertensives.

### BACKGROUND OF THE INVENTION

Renin-angiotensin system (RAS) plays a central role in the regulation of normal blood pressure and seems to be critically involved in hypertension development and maintenance as well as congestive heart failure. Angiotensin II (AII), an octapeptide hormone is produced mainly in the blood during the cleavage of angiotensin I by angiotensin converting enzyme (ACE) localized on the endothelium of blood vessels of lung, kidney, and many other organs, and is the end product of the RAS AII is a powerful arterial vasoconstricter that exerts its action by interacting with specific receptors present on cell membranes. One of the possible modes of controlling the RAS is angiotensin II receptor antagonism. Several peptide analogs of A II are known to inhibit the effect of this hormone by competitively blocking the receptors, but their experimental and clinical applications have been limited by the partial agonist activity and lack of oral absorption [M. Antonaccio. Clin. Exp. Hypertens. A4, 27-46 (1982); D. H. P. Streeten and G. H. Anderson, Jr. - Handbook of Hypertension, Clinical Pharmacology of Antihypertensive Drugs, ed. A. E. Doyle, Vol. 5, pp. 246-271, Elsevier Science Publisher, Amsterdam, The Netherlands, 1984].

Recently, several non-peptide compounds have been described as A II antagonists. Illustrative of such compounds are those disclosed in U.S. Patents 4,207,324; 4,340,598; 4,576,958; 4,582,847; and 4,880,804 in European Patent Applications 028,834; 245,637; 253,310; and 291,969; and in articles by A.T. Chiu, et al. [Eur. J. Pharm. Exp. Therap, 157, 13-21 (1988)] and by P.C. Wong, et al. [J. Pharm. Exp. Therap, 247, 1-7-(1988)]. All of the U.S. Patents, European Patent Applications 028,834 and 253,310 and the two articles disclose substituted imidazole compounds which are generally bonded through a lower alkyl bridge to a substituted phenyl. European Patent Application 245,637 discloses derivatives of 4,5,6,7-tetrahydro- 2H-imidazo[4,5-c]-pyridine-6-carboxylic acid and analogs thereof as antihypertensive agents.

None of the compounds disclosed in the above identified U.S. Patents, European Applications and articles are the substituted benzimidazole compounds of the invention.

### DETAILED DESCRIPTION OF THE INVENITON

This invention relates to novel substituted benzimidazole compounds and derivatives thereof which are useful as angiotensin II antagonists, as antihypertensives, in the treatment of congestive heart failure and in the treatment of elevated intraocular pressure. The compounds of this invention have the general formula:

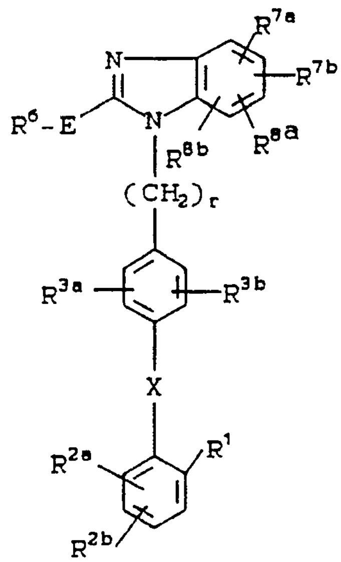

(I)

wherein:

$R^1$ is

(a) $-CO_2R^4$,
(b) $-SO_3R^5$,
(c) $-NHSO_2CF_3$,
(d) $-PO(OR^5)_2$,
(e) $-SO_2-NH-R^9$,
(f) $-CONHOR^5$.
(g) $-SO_2NH$-heteroaryl,
(h) $-CH_2SO_2NH$-hetroaryl,
(i) $-SO_2NHCOR^{2'}$,
(j) $-CH_2SO_2NHCOR^{2'}$,
(k) $-CONHSO_2R^{2'}$,
(l) $-CH_2CONHSO_2R^{2'}$,
(m) $-NHSO_2NHCOR^{2'}$,
(n) $-NHCONHSO_2R^{2'}$,
(o) $-SO_2NHCONHR^{2'}$,
(p) $-SO_2NHCN$,
(q) $-CONHOR^5$,

(r) 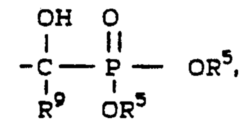,

(s) 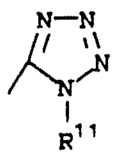,

(t) 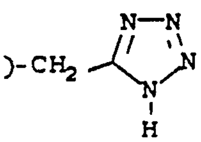,

(u) 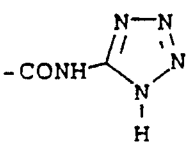,

(v) $-CONHNHSO_2CF_3$ ,

(w) 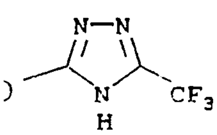

(x) 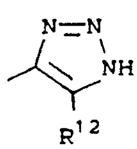 ;

(y) 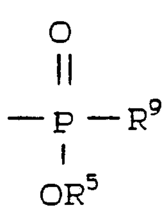

wherein:

heteroaryl is an unsubstituted, monosubstituted or disubstituted 5- or 6-membered aromatic ring which can optionally contain from 1 to 3 heteroatoms selected from the group consisting of O, N and S and wherein the substituents are members selected from the group consisting of -OH, -SH, $C_1$-$C_4$-alkyl, $C_1$-$C_4$-alkoxy, $-CF_3$, halo (Cl, Br, F, I), $-NO_2$, $-CO_2H$, $-CO_2$-$C_1$-$C_4$-alkyl, $-NH_2$, -NH($C_1$-$C_4$-alkyl) or -N($C_1$-$C_4$-alkyl)$_2$; $R^{2a}$ and $R^{2b}$ are independently H, halo (Cl, Br, I, F), $-NO_2$, $-NH_2$, $C_1$-$C_4$-alkylamino, $-SO_2NHR^9$, $CF_3$ $C_1$-$C_4$-alkyl, or $C_1$-$C_4$-alkoxy;

$R^{3a}$ is

(a) H,

(b) halo(Cl, Br, I, F)

(c) $C_1$-$C_6$-alkyl,
(d) $C_1$-$C_6$-alkoxy,
(e) $C_1$-$C_6$-alkoxyalkyl; $R^{3b}$ is
(a) H,
(b) halo (Cl, Br, I, F)
(c) $NO_2$,
(d) $C_1$-$C_6$-alkyl,
(e) $C_1$-$C_6$-acyloxy,
(f) $C_1$-$C_6$-cycloalkyl
(g) $C_1$-$C_6$-alkoxy,
(h) $-NHSO_2R^4$,
(i) hydroxy $C_1$-$C_4$-alkyl,
(j) aryl $C_1$-$C_4$-alkyl
(k) $C_1$-$C_4$-alkylthio
(l) $C_1$-$C_4$-alkyl sulfinyl
(m) $C_1$-$C_4$-alkyl sulfonyl
(n) $NH_2$
(o) $C_1$-$C_4$-alkylamino
(p) $C_1$-$C_4$-dialkylamino
(q) fluoro $C_1$-$C_4$-alkyl
(r) $-SO_2-NHR^9$
(s) aryl or,
(t) furyl; wherein aryl is phenyl or naphthyl optionally substituted with one or two substituents selected from the group consisting of halo(Cl, Br, I, F) $C_1$-$C_4$-alkyl, $C_1$-$C_4$-alkoxy, $NO_2$, $CF_3$, $C_1$-$C_4$-alkylthio, OH or $NH_2$;

$R^4$ is H, straight chain or branched $C_1$-$C_6$ alkyl, aryl, or $-CH_2$-aryl where aryl is as defined above;
$R^{4a}$ is $C_1$-$C_6$-alkyl, aryl, or $-CH_2$-aryl where aryl is as defined above;

$R^5$ is H, 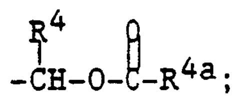;

E is a single bond, $NR^{13}(CH_2)_s$-, $-S(O)_x(CH_2)_s$-where x is 0 to 2 and s is 0 to 5, -CH(OH)-, -O-, -CO-;
$R^6$ is
(a) aryl as defined above optionally substituted with 1 or 2 substituents selected from the group consisting of halo (Cl, Br, I, F) -O-$C_1$-$C_4$-alkyl, $C_1$-$C_4$-alkyl, $-NO_2$, $-CF_3$, $-SO_2NR^9R^{10}$, -S-$C_1$-$C_4$-alkyl, -OH, $-NH_2$, $C_3$-$C_7$-cycloalkyl, $C_3$-$C_{10}$-alkenyl;
(b) straight chain or branched $C_1$-$C_6$-alkyl, $C_2$-$C_5$-alkenyl or $C_2$-$C_5$-alkynyl each of which can be optionally substituted with a substituent selected from the group consisting of aryl as defined above, $C_3$-$C_7$-cycloalkyl, halo (Cl, Br, I, F) -OH, $-NH_2$, $-NH(C_1$-$C_4$-alkyl), $-N(C_1$-$C_4$-alkyl$)_2$, $-NH-SO_2R^4$, $-COOR^4$, $-SO_2NHR^9$; or
(c) an unsubstituted, monosubstituted or disubstituted aromatic 5 or 6 membered cyclic ring which can contain one or two members selected from the group consisting of N, O, S, and wherein the substituents are members selected from the group consisting of -OH, -SH, $C_1$-$C_4$-alkyl, $C_1$-$C_4$-alkyloxy $-CF_3$, halo (Cl, Br, I, F), or $NO_2$; $R^{7a}$ and $R^{7b}$ are independently
(a) H,
(b) straight chain or branched $C_1$-$C_6$-alkyl, $C_2$-$C_6$-alkenyl or $C_2$-$C_6$-alkynyl,
(c) halo(Cl, Br, I, F)
(d) perfluoro-$C_1$-$C_4$-alkyl,
(e) $C_3$-$C_5$-cycloalkyl, or
(f) when $R^{7a}$ and $R^{7b}$ are bonded to adjacent carbon atoms, they can be joined to form an aryl or heteroaryl ring wherein the aryl is as defined above and the heteroaryl is as defined below; $R^{8a}$ and $R^{8b}$ are independently
(a) H,
(b) aryl-$C_1$-$C_4$-alkyl,
(c) heteroaryl-$C_1$-$C_4$-alkyl,
(d) $C_1$-$C_4$-alkyl, $C_2$-$C_6$-alkenyl or $C_2$-$C_6$ alkynyl each of which can be optionally substituted with a

substituent selected from the group consisting of -OH, -SH, $-NH_2$, amidino, guanidino, cyano, $C_1$-$C_4$-alkoxy, $C_1$-$C_4$-alkylthio, ($C_1$-$C_4$-alkyl)amino, di-($C_1$-$C_4$-alkyl)amino, $-COOR^4$, $-CON(R^4)_2$, $-O-COR^4$, $-N(R^4-COR^4$,$-CON(CH_2-CH_2)_2Z$, -NHCOO($C_1$-$C_4$-alkyl), $-S(O)_xR^{21}$, $-CONHSO_2R^{21}$, $-SO_2NHCOR^{21}$, $-SO_2NH$-heteroaryl as defined below, tetrazol-5-yl, $-PO(OR^4)2$, $-PO(OR^4)R^9$ or heteroaryl as defined below;

(e) -CO-aryl,
(f) $C_3$-$C_7$-cycloalkyl,
(g) halo (Cl, Br, I, F),
(h) -OH,
(i) $-OR^{21}$,
(j) -perfluoro-$C_1$-$C_4$-alkyl,
(k) -SH,
(l) $-S(O)_x-R^{21}$ where x is as defined above,
(m) -CHO,
(n) $-CO_2H$,
(o) $-CO_2$-$C_1$-$C_4$-alkyl,
(p) $-SO_3H$,
(q) $-NH_2$,
(r) -NH($C_1$-$C_4$-alkyl),
(s) -N($C_1$-$C_4$-alkyl$)_2$,
(t) $-NHCO_2$-$C_1$-$C_4$-alkyl,
(u) $-SO_2NHR^9$,
(v) $-SO_2NR^9R^{10}$,
(w) $-NO_2$,
(x) $-NH-SO_2R^{21}$,
(y) aryl,
(z) heteroaryl,
(aa) -NH-($C_3$-$C_7$-cycloalkyl),
(bb)

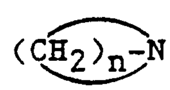

where n is 4 to 6,
(cc) -CN,
(dd) tetrazol-5-yl,
(ee) $-CONHSO_2R^{21}$,
(ff) $-SO_2NHCOR^{21}$,
(gg) $-SO_2NH$-heteroaryl,
(hh) $-N(R^4)-CON(R^4)-R^{21}$,
(ii) $-PO(OR^4)_2$,
(jj) $-PO(OR^4)R^9$,
(kk) $-CON(CH_2-CH_2)_2$ Z, where Z is as defined below,
(ll) $-N(R^4)-COOR^{21}$,
(mm)

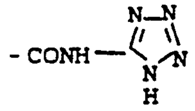 ,

(nn) $-N(CH_2-CH_2)_2-Z$ where Z is as defined below,

(oo) -NHCO-heteroaryl, wherein aryl is defined above and heteroaryl is a 5 or 6 membered heterocyclic ring containing up to three heteroatoms selected from the group consisting of O, N, or S wherein S may be in the form of sulfoxide or sulfone and wherein the heteroaryl may be substituted with halo (F, Cl, Br, I), $C_1$-$C_4$-alkyl, $C_1$-$C_4$-alkoxy, $NO_2$, OH, COOH, COO($C_1$-$C_4$-alkyl), $N(R^4)_2$,

$R^9$ is H, $C_1$-$C_5$-alkyl, aryl or $-CH_2$-aryl;

$R^{10}$ is H, $C_1$-$C_4$-alkyl;
$R^{11}$ is H, $C_1$-$C_6$-alkyl, $C_2$-$C_4$-alkenyl, $C_1$-$C_4$-alkoxy, or $-CH_2-C_6H_4R^{20}$;
$R^{12}$ is -CN, $-NO_2$ or $-CO_2R^4$; $R^{13}$ is H, -CO($C_1$-$C_4$-alkyl), $C_1$-$C_6$-alkyl, allyl, $C_3$-$C_6$-cycloalkyl, phenyl or benzyl;
$R^{14}$ is H, $C_1$-$C_8$-alkyl, $C_1$-$C_8$-perfluoroalkyl, $C_3$-$C_6$-cycloalkyl, phenyl or benzyl;
$R^{15}$ is H, $C_1$-$C_6$-alkyl;
$R^{16}$ is H, $C_1$-$C_6$-alkyl, $C_3$-$C_6$-cycloalkyl, phenyl or benzyl;
$R^{17}$ is $-NR^9R^{10}$, $-OR^{10}$, $-NHCONH_2$, $-NHCSNH_2$,

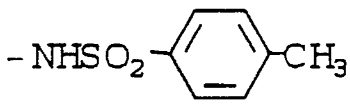 or 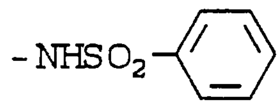 ;

$R^{18}$ and $R^{19}$ are independently $C_1$-$C_4$-alkyl or taken together are $-(CH_2)_q-$where q is 2 or 3;
$R^{20}$ is H, $-NO_2$, $-NH_2$, -OH or $-OCH_3$;
$R^{21}$ is

(a) aryl as defined above,
(b) heteroaryl as first defined above,
(c) $C_3$-$C_7$-cycloalkyl,
(d) perfluoro-$C_1$-$C_4$-alkyl,
(e) $C_1$-$C_4$-alkyl optionally substituted with a substituent selected from the group consisting of aryl as defined above, heteroaryl as defined above, -OH, -SH, $C_1$-$C_4$-alkyl, -O($C_1$-$C_4$-alkyl), S($C_1$-$C_4$-alkyl), $-CF_3$, halo(Cl, Br, F, I), $-NO_2$, $-CO_2H$, $CO_2$-$C_1$-$C_4$-alkyl, $-NH_2$, -NH($C_1$-$C_4$-alkyl), -N($C_1$-$C_4$-alkyl)$_2$, $-PO_3H$, -PO(OH)(O-$C_1$-$C_4$-alkyl); X is absent or is

(a) a carbon-carbon single bond,
(b) -CO-,
(c) -O-,
(d) -S-,

(e) 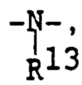

(f) 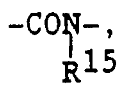

(g) 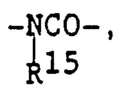

(h) $-OCH_2-$,
(i) $-CH_2O-$
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,
(p) -CH=CH-,
(q) -CF=CF-,
(r) -CH=CF-,
(s) -CF=CH-,
(t) $-CH_2CH_2-$,
(u) $-CF_2CF_2-$,
(v) 1,1 and 1,2-disubstituted cyclopropyl,

(w) 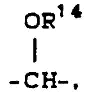 ,

(x) 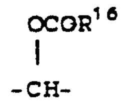

(y) 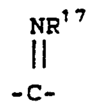 , or

(z) 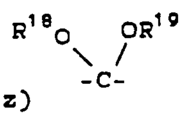 ;

Z is 0, $NR^{13}$ or S;
r is 1 or 2; and,
the pharmaceutically acceptable salts thereof.

One embodiment of the compounds of Formula (I) are those wherein:
$R^1$ is
(a) -COOH,
(b) $-NH-SO_2CF_3$,
(c)

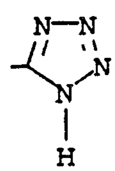 ,

(d) $-CONHSO_2R^{2'}$,
(e) $-SO_2NHCOR^{2'}$,

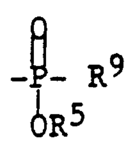

(g) $-SO_2NH$-heteroaryl, $R^{2a}$ is H;
$R^{2b}$ is H, F, Cl, $CF_3$ or $C_1$-$C_4$-alkyl;
$R^{3a}$ is H;
$R^{3b}$ is H, F, Cl, $CF_3$, $C_1$-$C_4$-alkyl, $C_5$-$C_6$-cycloalkyl, $-COOCH_3$, $-COOC_2H_5$, $-SO_2-CH_3$, $NO_2$, $-N(C_1-C_4\text{-alkyl})_2$ or $-NH-SO_2CH_3$;
E is a single bond, -O- or -S-;
$R^6$ is
(a) $C_1$-$C_5$ alkyl optionally substituted with a substituent selected from the group consisting of methyl, ethyl, Cl, $CF_3$, $CCl_3$, $-O-CH_3$, $-OC_2H_5$, $-S-CH_3$, $-S-C_2H_5$ or phenyl;
(b) $C_1$-$C_5$-alkenyl or $C_1$-$C_5$-alkynyl;
(c) aryl as defined above optionally substituted with a substituent selected from the group consisting of halo(Cl, F, Br, I), $-CF_3$, $-NO_2$, -OH, $-NH_2$, $-S-CH_3$, $-S-C_2H_5$, $-SO_2NH_2$ $-O-CH_3$; or,
(d) a heteroaryl which is a member selected from the group consisting of 2-pyridyl, 4-pyridyl, 2-pyrimidyl, 6-pyrimidyl, imidazoyl, thiazolyl, imidolyl, thienyl, or furyl; $R^{7a}$ and $R^{7b}$ are each H or when $R^{7a}$ and $R^{7b}$ are bonded to adjacent carbon atoms, they can be joined to form a phenyl ring;

$R^{8a}$ and $R^{8b}$ are independently

(a) H,
(b) $C_1$-$C_4$-alkyl,
(c) $C_1$-$C_4$-alkenyl,
(d) -OH,
(e) $-NO_2$,
(f) $-NH_2$,

O

(g) -NH- C -,
(h) -$C_1$- $C_4$-alkoxy,

O

(i) -NH-C-O-$C_1$-$C_4$-alkyl,
(j) -N($C_1$-$C_4$-alkyl$)_2$,
(k) halo(Cl, F, Br),
(l) $-CF_3$,
(m) $-CO_2$-$C_1$-$C_4$-alkyl,
(n) -COOH,
(o) $-CO_2$-OH,
(p) -CO-$C_1$-$C_4$-alkyl,
(q) -CO-aryl as defined above,
(r) -thio-$C_1$-$C_4$-alkyl
(s) $-SO_2$-NH-$C_1$-$C_4$-alkyl,
(t) $-SO_2$-NH-aryl as defined above,
(u) $-NH-SO_2CO_3$,
(v) aryl as defined above: X is aC-C single bond or -CO-; and,

r is one.

Another embodiment of the compounds of Formula (I) are those wherein:

E is a single bond or -S-;
r is one,
$R^{2a}$, $R^{2b}$, $R^{3a}$ and $R^{3b}$ are each H;
$R^6$ is n-propyl, n-butyl, $-CH_2Cl$, $-CH_3$, $-CH_2CH_3$, $-CH_2$-S-$CH_3$,

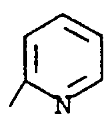 ;

$R^{7a}$ and $R^{7b}$ are each H:
$R^{8a}$ is H, $-CH_3$, -Cl, $-COOCH_3$, -F, $-CH_2OH$,

O

phenyl- C -, $-OCH_3$, $-N(CH_3)_2$, -NH-CO-isopropyl, $-CH_2$-O-$CONH_2$, $-CF_3$, $-N(CH_3)_2$, isopropyl;
$R^{8b}$ is H, $-CH_3$, -Cl, $-COOCH_3$, F, $-CH_2OH$,

O

phenyl- C -, $-OCH_3$, $-N(CH_3)_2$;
$R^1$ is -COOH, $-CONHSO_2$-phenyl,

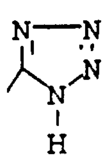 or $-NH-SO_2-CF_3$ and,

X is a single bond or -CO-.

A further embodiment of the compounds of Formula (1) are those wherein:

$R^1$ is -COOH;

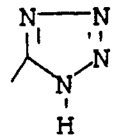 or $-NH-SO_2CF_3$

$R^{2a}$, $R^{2b}$, $R^{3a}$ and $R^{3b}$ are each H;
E is as defined above;
r is one;
$R^6$ is n-propyl, n-butyl;
$R^{7a}$ and $R^{7b}$ are each H;
$R^{8a}$ is H, $-CO_3$, Cl, $-COOCH_3$, $-NH-COOCH_3$, $-NH-CO-OC_2H_5$, $-CH_2OH$, $NO_2$;
$R^{8b}$ is H, $-CH_3$, Cl, $C_2H_5$, $-N(CH_3)_2$; or,
$R^{7a}$ and $R^{7b}$ are joined together to form a cyclic $C_4H_4$-ring.

One illustrative class of the compounds of the invention are those such as:

(1) 2-propyl-1-(2′-carboxybiphen-4-yl)methylbenzimidazole;
(2) 2-propyl-1-(4-(2′-carboxybenzoyl)benzylbenzimidazole;
(3) 2-butyl-1-(2′-carboxybiphen-4-yl)methylbenzimidazole;
(4) 2-butyl-4-methyl-1-(2′-carboxybiphen-4-yl)methylbenzimidazole;
(5) 2-butyl-7-methyl-1-(2′-carboxybiphen-4-yl)methylbenzimidazole;
(6) 2-butyl-4,5-dimethyl-1-(2′-carboxybiphen-4-yl)methylbenzimidazole;
(7) 2-butyl-5,6-dimethyl-1-(2′-carboxybiphen-4-yl)methylbenzimidazole;
(8) 2-butyl-5,6-dichloro-1-(2′-carboxybiphen-4-yl)methylbenzimidazole;
(9) 2-butyl-1-(2′-carboxybiphen-4-yl)methyl-1H-naphth[2,3-d]imidazole;
(10) 2-butyl-5-carbomethoxy-1-(2′-carboxybiphen-4-yl)methylbenzimidazole;
(11) 2-butyl-1′-(2′-(tetrazol-5-yl)biphen-4-yl)methylbenzimidazole;
(12) 2-butyl-4-methyl-1-(2′-(tetrazol-5-yl)-biphen-4-yl)methylbenzimidazole;
(13) 2-propyl-4-methyl-1-(2′-(tetrazol-5-yl)-biphen-4-yl)methylbenzimidazole;
(14) 2-butyl-5-carbomethoxy-1-(2′-(tetrazol-5-yl)biphen-4-yl)methylbenzimidazole;
(15) 2-butyl-7-hydroxymethyl-1-(2′-(tetrazol-5-yl)biphen-4yl)methylbenzimidazole;
(16) 2-butyl-5-hydroxymethyl-1-(2′-(tetrazol-5-yl)biphen-4-yl)methylbenzimidazole;
(17) 4,6-dimethyl-2-propyl-1-(2′-(tetrazol-5-yl)biphen-4-yl)methylbenzimidazole;
(18) 4,6-dimethyl-2-ethyl-1-(2′-(tetrazol-5-yl)biphen-4-yl)methylbenzimidazole;
(19) 4,6-dimethyl-2-ethyl-5-fluoro-(2′-(tetrazol-5-yl)biphen-4-yl)methylbenzimidazole;
(20) 2-cyclopropyl-4,6-dimethyl-1-(2′-(tetrazol-5-yl)biphen-4-yl)methylbenzimidazole;
(21) 2-cyclopropyl-4,6-dimethyl-5-fluoro-1-(2′-(tetrazol-5-yl)biphen-4-yl )methylbenzimidazole;
(22) 2-cyclopropyl-4,6-dimethyl-5-fluoro-1-(4′-fluoro-2′-(tetrazol-5-yl)biphen-4-yl)methylbenzimidazole;
(23) 2-cyclopropyl-4,6-dimethyl-1-(4′-dimethylamino-2′-(tetrazol-5-yl)biphen-4-yl)methyl-5-fluorobenzimidazole;
(24) 4,6-dimethyl-1-(2′-(tetrazol-5-yl)biphen-4-yl)methyl-2-(3,3,3-trifluoropropyl)benzimidazole;
(25) 4,6-dimethyl-2-pentafluoroethyl-2-(2′-(tetrazol-5-yl)biphen-4-yl)methylbenzimidazole;
(26) 2-(3-butynyl)-4,6-dimethyl-1-(2′-(tetrazol-5-yl)biphen-4-yl)methylbenzimidazole;
(27) 6-dimethylamino-2-ethyl-4-methyl-1-(2′-(tetrazol-5-yl)biphen-4-yl)methylbenzimidazole;
(28) 2-ethyl-4-methyl-6-(morpholin-4-yl)-1-(2′-(tetrazol-5-yl)biphen-4-yl)methylbenzimidazole;
(29) 2-ethyl-4-methyl-6-methylamino-1-(2′-(tetrazol-5-yl)biphen-4-yl)methylbenzimidazole;
(30) 6-carboxy-2-ethyl-4-methyl-1-(2′-(tetrazol-5-yl)biphen-4-yl)methylbenzimidazole;
(31) 6-Carbomethoxy-2-ethyl-4-methyl-1-(2′-(tetrazol-5-yl)biphen-4-yl)methylbenzimidazole;
(32) 2-ethyl-6-hydroxymethyl-4-methyl-1-(2′-(tetrazol-5-yl)biphen-4-yl)methylbenzimidazole;
(33) 1-(2′-(N-acetyl)sulfonamidomethylbiphen-4-yl)methyl-4,6-dimethyl-2-ethylbenzimidazole;
(34) 1-(2′-(N-acetyl)sulfonamidomethylbiphen-4-yl)methyl-2-cyclopropyl-4,6-dimethylbenzimidazole;
(35) 4,6-dimethyl-2-ethyl-1-(2′-(N-phenylsulfonyl)carboxamidobiphen-4-yl)methylbenzimidazole;
(36) 2-cyclopropyl-4,6-dimethyl-1-(2′-(N-phenylsulfonyl)carboxamidobiphen-4-yl)methylbenzimidazole;
(37) 4,6-dimethyl-2-ethyl-1-(2′-(N-pyrimidin-2-yl)sulfonamidobiphen-4-yl)methylbenzimidazole;
(38) 2-cyclopropyl-4,6-dimethyl-1-(2′(N-pyrimidin-2-yl)sulfonamidobiphen-4-yl)methylbenzimidazole; and,
(39) 2-butyl-1-(2′-(N-phenylsulfonyl)carboxamidobiphen-4-yl)methylbenzimidazole.

The compounds of Formula (I) can be synthesized using the reactions and techniques described hereinbelow. The reactions are performed in a solvent appropriate to the reagents and materials employed and suitable for the transformation being effected. It is understood by those skilled in the art of organic synthesis that the functionality present on the benzimidazole and other parts of the structure should be consistent with the chemical transformations proposed. Depending upon the reactions and techniques employed, this may involve changing the order of synthetic steps, use of required protecting groups followed by deprotection, and activation of the benzylic position of the alkylating agents used to enable alkylation at the nitrogen on the imidazole part of benzimidazoles.

## REACTION SCHEME 1

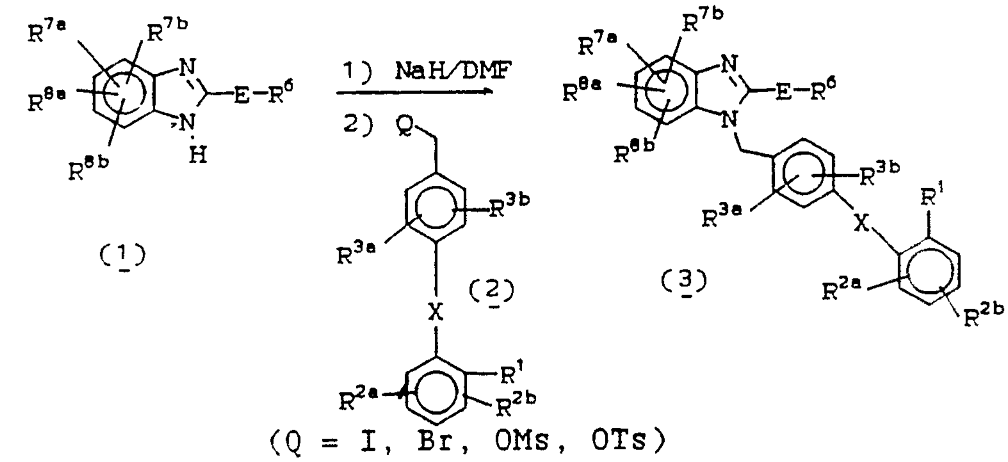

(1) (2) (3)

(Q = I, Br, OMs, OTs)

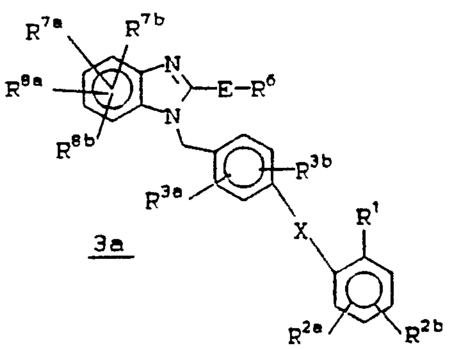
3a

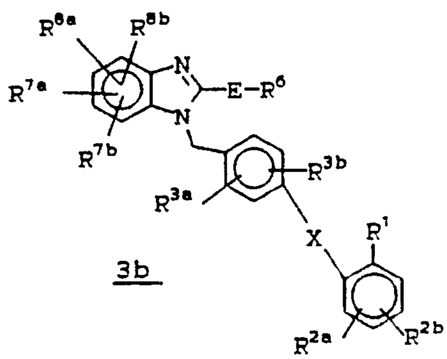
3b

As shown in Reaction Scheme 1, compounds of Formula (3) can be prepared by carrying-out direct alkylation of alkali-metal salt of benzimidazole (1) (preparation of benzimidazoles are described in Reaction Schemes 3-6) using appropriately protected benzyl halide, tosylate (OTs) or mesylate (OMs) derivatives (2). The salt is prepared preferably using MH (where M is lithium, sodium or potassium) in anhydrous dimethylformamide (DMF), or by treating it with metal alkoxoide such as sodium or potassium methoxide,

ethoxide or t-butoxide in an appropriate alcohol such as methanol, ethanol or t-butanol as the solvent. The alkylation is generally carried-out by dissolving the metal salt of benzimidazole in a dipolar aprotic solvent such as DMF or dimethylsulfoxide (DMSO) and reacting it with the alkylating agent at 20 °C to reflux temperature of the solvent for 1-24 hours.

If substituents on the benzene ring result in an unsymmetrical benzimidazole, the alkylation may produce a mixture of two regioisomers as products arising from $N^1$ and $N^3$ alkylation. These regioisomers possess distinct physico-chemical and biological properties and in most cases can be separated and purified by using conventional separation techniques such as chromatography (flash column chromatography, medium-pressure liquid chromatography, high pressure liquid chromatography (HPLC) and/or crystallization. In those cases where separation of regioisomers is difficult by conventional techniques, the mixture can be transformed into suitable derivatives that can be separated by usual separation methods. The structural assignments of the isomers can be made using proton NMR, Nuclear Overhauser Effect (NOE) experiments or X-ray crystallography.

## REACTION SCHEME 2

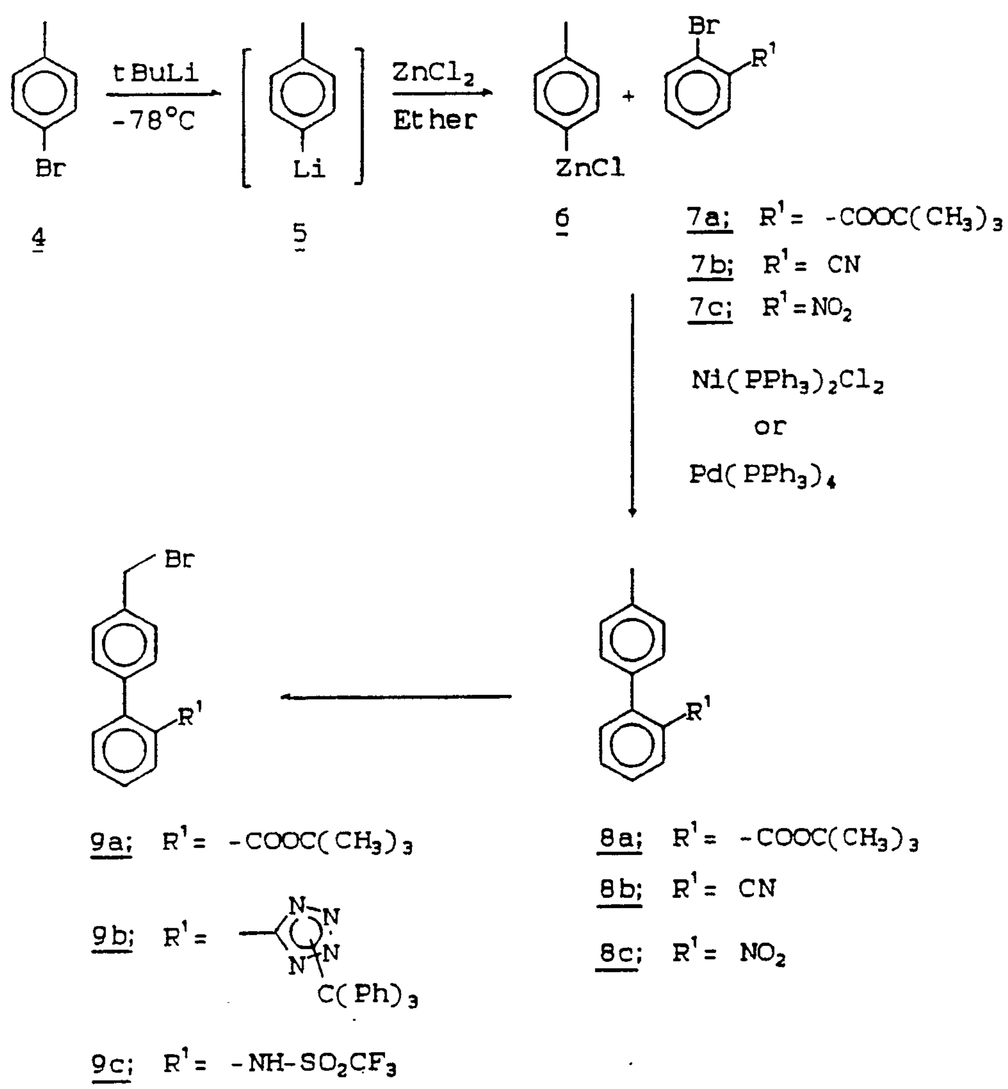


4

5

6

7a; $R^1$ = $-COOC(CH_3)_3$

7b; $R^1$ = CN

7c; $R^1$ = $NO_2$

8a; $R^1$ = $-COOC(CH_3)_3$

8b; $R^1$ = CN

8c; $R^1$ = $NO_2$

9a; $R^1$ = $-COOC(CH_3)_3$

9b; $R^1$ =

9c; $R^1$ = $-NH-SO_2CF_3$

The benzyl halides (2) including the more preferred alkylating agents (9a and 9b, Reaction Scheme 2) can be prepared as described in European Patent Applications 253,310 and 291,969 and the references

cited therein. However, a preferred method to prepare the biphenyl precursors 8a, 8b and 8c using Ni(O) or Pd(O) catalyzed cross-coupling reaction [E. Negishi, T. Takahashi, and A. O. King, Org. Synthesis, 66, 67 (1987)] is outlined in Reaction Scheme 2. As shown in Reaction Scheme 2, treatment of 4-bromo toluene (4) with t-BuLi, followed by the addition of a solution of $ZnCl_2$, produces the organo-zinc compound (6). Compound (6) is then coupled with 7a or 7b in the presence of $Ni(PPh_3)Cl_2$ catalyst to produce the desired biphenyl compound 8a or 8b ($PPh_3$ = triphenylphosphine). Similarily, 1-iodo-2-nitro-benzene (7c) is coupled with organo-zinc compound 6 in the presence of $Pd(PPh_3)_4$ catalyst [prepared by treating $Cl_2Pd(PPh_3)_2$ with $(i\text{-}Bu)_2$ AlH (2 equiv.)] to give the biphenyl compound 8c. These precursors, 8a, 8b and 8c, are then transformed into halomethylbiphenyl derivatives 9a, 9b and 9c, respectively, according to procedures described in European Patent Applications 253,310 and 291,969.

## REACTION SCHEME 3:

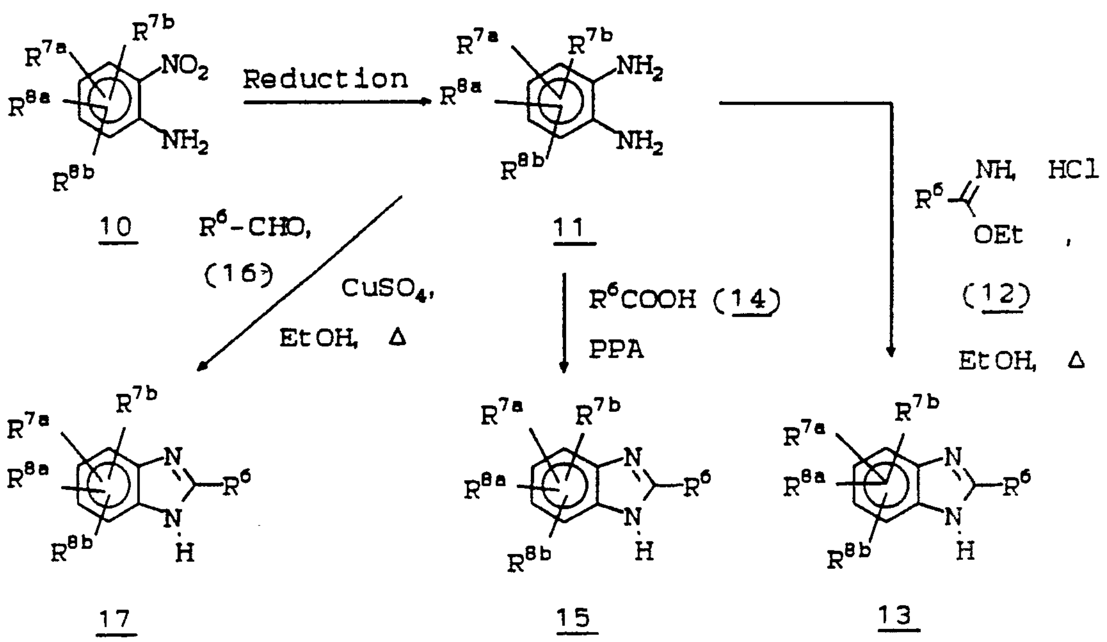


The starting benzimidazoles can be readily prepared by any of the standard procedures described in the litrature [P. N. Preston, Chemistry of Heterocyclic Compounds, Vol. 40, part I, pp. 1-286 (1981) and references cited therein]. Several alternative routes to obtain benzimidazoles are outlined in Reaction Scheme 3. The most widely used starting material, o-phenylenediamines (11), can be readily prepared from the corresponding o-nitroaniline (10) using standard reductive procedures such as metal-acid reduction or catalytic reduction. The substituted or unsubstituted (11) can then be treated with an appropriate imidate hydrochloride (12) to form corresponding benzimidazoles (13). Alternatively, the reaction of carboxylic acids (14) with o-phenylenediamines in the presence of polyphosphoric acid (PPA) is also effective in producing benzimidazoles (15). Benzimidazoles (17) can also be prepared from o-phenylenediamines and aldehyde (16) using cupric salt as an oxidant [R. Weidenhagen, Chem. Ber., 69, 2263 (1936)].

REACTION SCHEME 4

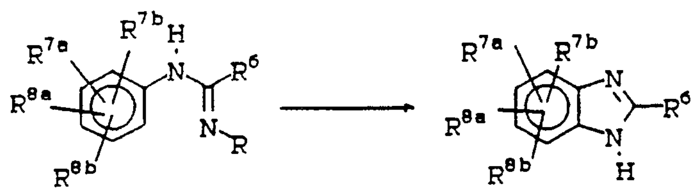

(R= H, OH, Cl) (R$^6$= aryl, heteroaryl)

18 19

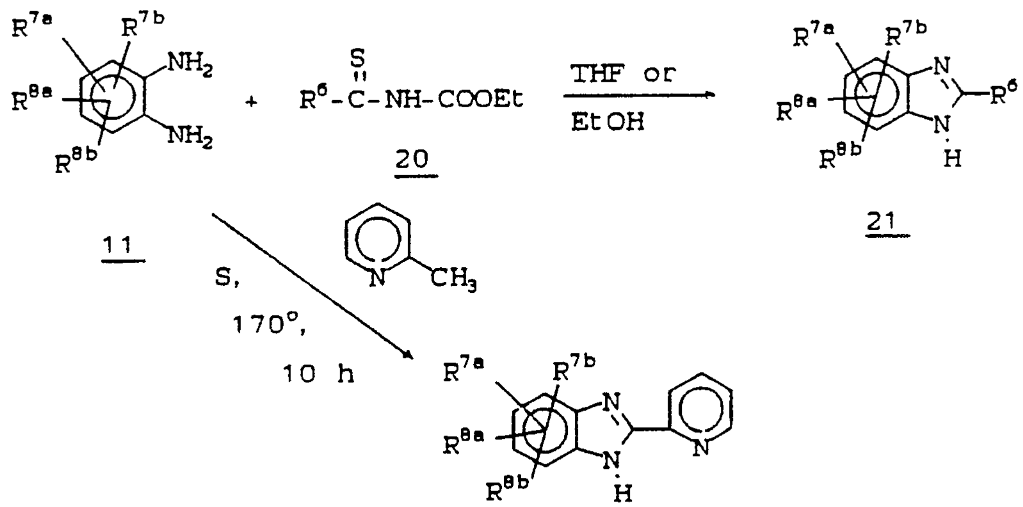


20

11

21

22

Although some benzimidazoles having aryl and heteroaryl groups at the 2 position can be prepared using the methods described in Reaction Scheme 3, Reaction Scheme 4 outlines methods which are more suitable for the synthesis of this class of compounds. N′-aryl-N-hydroxyamidines (18; R = OH) are cyclized under mild conditions using benzenesulfonylchloride in pyridine or triethylamine to give 19 in good yield [M. W. Partridge and H. A. Turner, J Chem. Soc., 2086 (1958)]. Parent amidines (18; R = H) can also be oxidized with sodium hypochlorite under basic condition to form 19 [V. J. Grenda, R. E. Jones, G. Gal and M. Sletzinger, J. Org. Chem., 30, 259, (1965)].

Alternatively, as shown in Reaction Scheme 4, o-phenylenediamines (11) can be reacted with N-ethoxycarbonylthioamides (20) to give 2-substituted benzimidazoles (21) in excellent yields. This method avoids the use of acidic catalysts. The reagents (20) are easily obtained in one step from ethoxycarbonyl isothiocyanate and simple aromatic or heterocyclic compounds or alkylmagnesium halides [B. George and E. P. Papadopoulos., J. Org. Chem., 41, 3233(1976); E. P. Papadopoulos., J. Org. Chem., 41, 962(1976)]. Heterocyclic compounds containing reactive methyl groups (e.g., 2-picoline) can also be reacted with o-phenylenediamines in the presence of sulfur at elevated temperatures to give 2-heteroaryl benzimidazoles (22).

## REACTION SCHEME 5

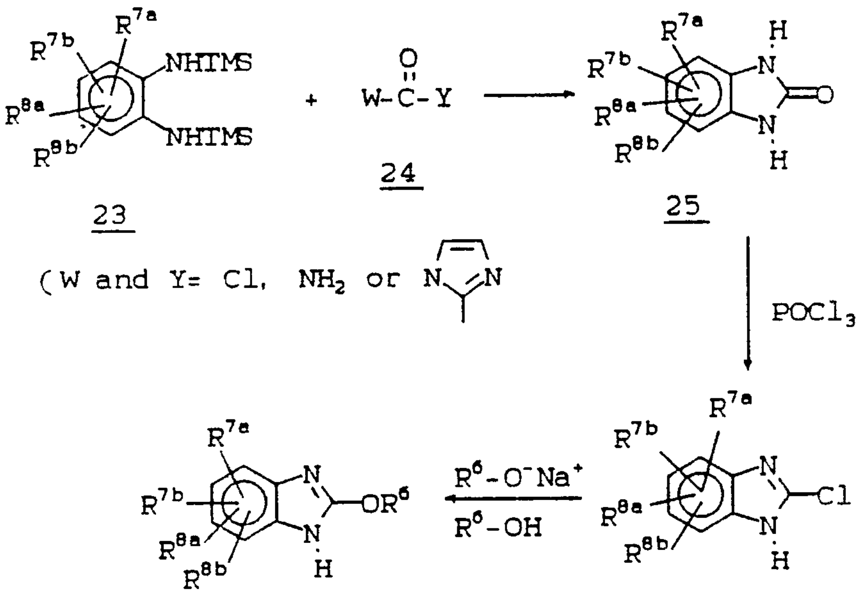


23 24 25 26 27

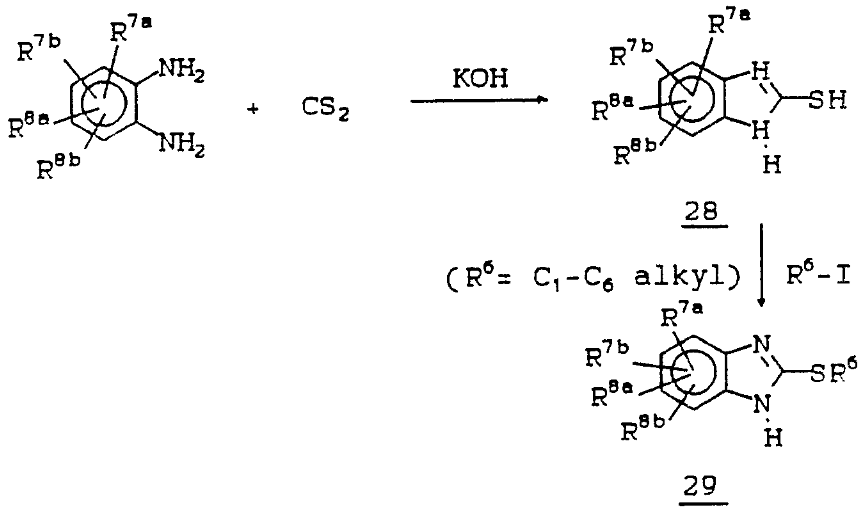


28 29

As outlined in Reaction Scheme 5, benzimidazoles containing 2-alkoxy and thioalkyl substituents (27 and 29) can be prepared from the corresponding benzimidazolones (25) or benzimidazolethiones (28). Benzimidazolones are conveniently prepared from o-phenylenediamines and phosgene or urea [K. Hofmann, "Imidazole and its Derivatives, Part 1," Wiley-Interscience, New York, 1953, pp. 285-291]. Carbonate esters, diethylpyrocarbonate, N,N-carbonyldiimidazole and N,N-diethylcarbamyl chloride may also be used in this reaction. The reaction of phosgene is apparently facilitated by the use of N,N′-bis trimethylsilyl (TMS) derivative (23) instead of the parent diamine [L. Birkhofer, H. P. Kuhlthau, and A. Ritter, Chem. Ber., 93, 2810 (1960)].

## REACTION SCHEME 6

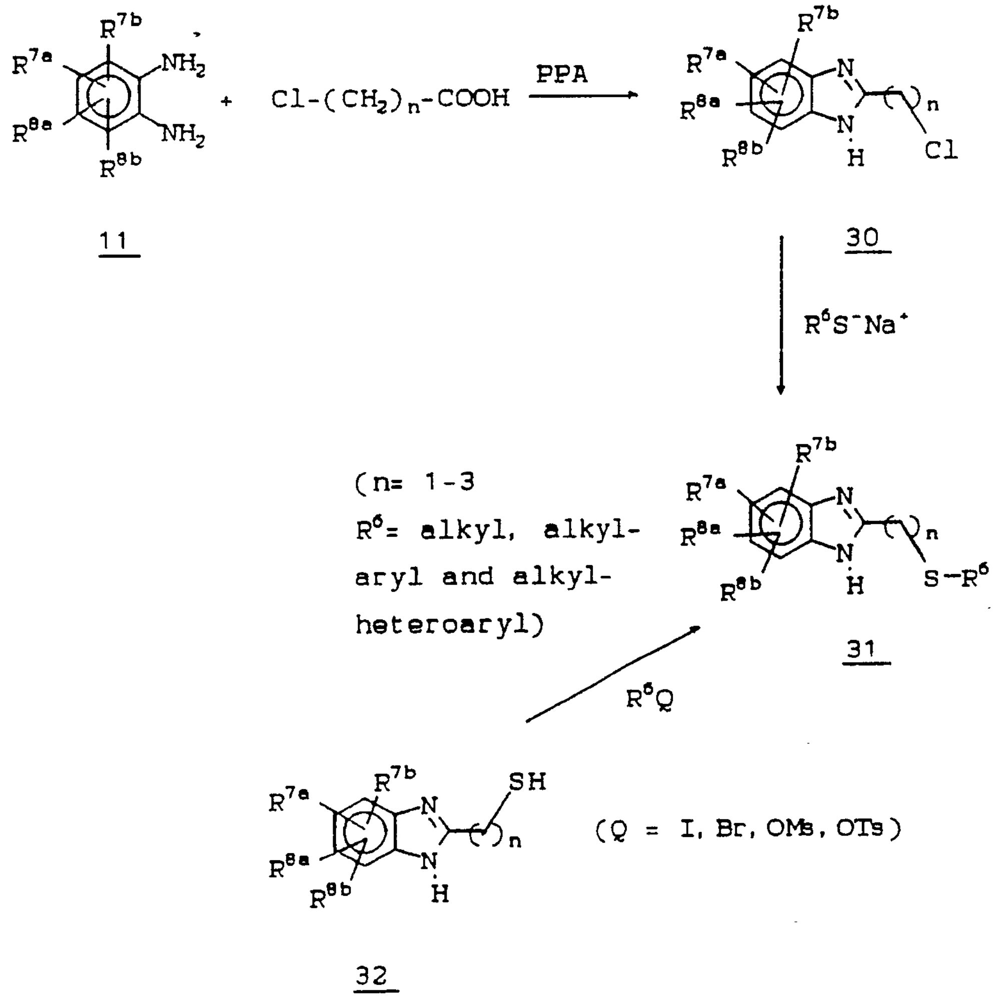


11 30 31 32

As described in Reaction Scheme 6, 2-alkylthioalkyl substituted benzimidazoles (31) can be prepared from the reaction of RS-M (where M is sodium, potassium or lithium) with 2-chloroalkyl benzimidazoles (30). 2-Chloroalkyl benzimidazoles (30) can be conveniently prepared from the diamines and the chloroalkyl carboxylic acids using PPA [W. Knobloch, Chem. Ber., 91, 2557 (1958)]. Alternatively, compound 31 can also be prepared from the readily available 2-thioalkyl derivative (32) [E. S. Milner. S. Snyder, and M. M. Joullie, J. Chem. Soc., 4151 (1964)].

Compounds of formula I where $R^1$ is $-CONHSO_2R^{21}$ (where $R^{21}$ = alkyl, aryl or heteroaryl) may be prepared from the corresponding carboxylic acid derivatives 3a as outlined in Scheme 7. The carboxylic acid 3a, obtained as described in Scheme 1, can be converted into the corresponding acid chloride by treatment with refluxing thionyl chloride or preferably with oxalylchloride and a catalytic amount of dimethylformamide at low temperature [A. W. Burgstahler, L. O. Weigel, and C. G. Shaefer-Synthesis, 767, (1976)]. The acid chloride then can be treated with the alkali metal salt of $R^{21}SO_2NH_2$ to form the desired acylsulfonamide 33. Alternatively, these acylsulfonamides may be also prepared from the carboxylic acids using N,N-diphenylcarbamoyl anhydride intermediates [F. J. Brown et al - European Patent Application, EP 199543; K. L. Shepard and W. Halczenko- J. Het. Chem., 16, 321 (1979)]. Preferably the carboxylic acids can be converted into acyl-imidazole intermediates, which then can be treated with an appropriate aryl or

alkylsulfonamide and diazabicycloundecane (DBU) to give the desired acylsulfonamide 33 [J. T. Drummond and G. Johnson -Tetra. Lett., 29, 1653 (1988)].

Compounds of formula I where R' is $-SO_2NHCOR^{21}$ may be prepared as outlined in Scheme 8. The nitro compound 8c (prepared as described in Scheme 2) can be reduced to the corresponding amino compound and converted into aromatic diazonium chloride salt, which then can be reacted with

## Scheme 7

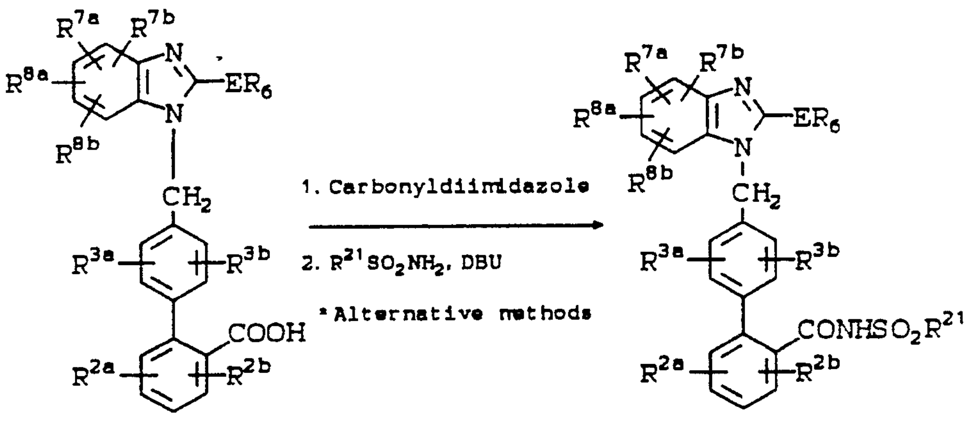


3a 33

*Alternative Methods:

a) (i) $SOCl_2$, reflux (ii) $R^{21}SO_2NH^-M^+$ (where M is Na or Li)

b) (i) $(COCl)_2$-DMF, -20°C (ii) $R^{21}SO_2NH^-M^+$

c) (i) N(N,N-Diphenylcarbamoyl)pyridinium chloride/ Aq. NaOH (ii) $R^{21}SO_2NH^-M^+$.

Scheme 8

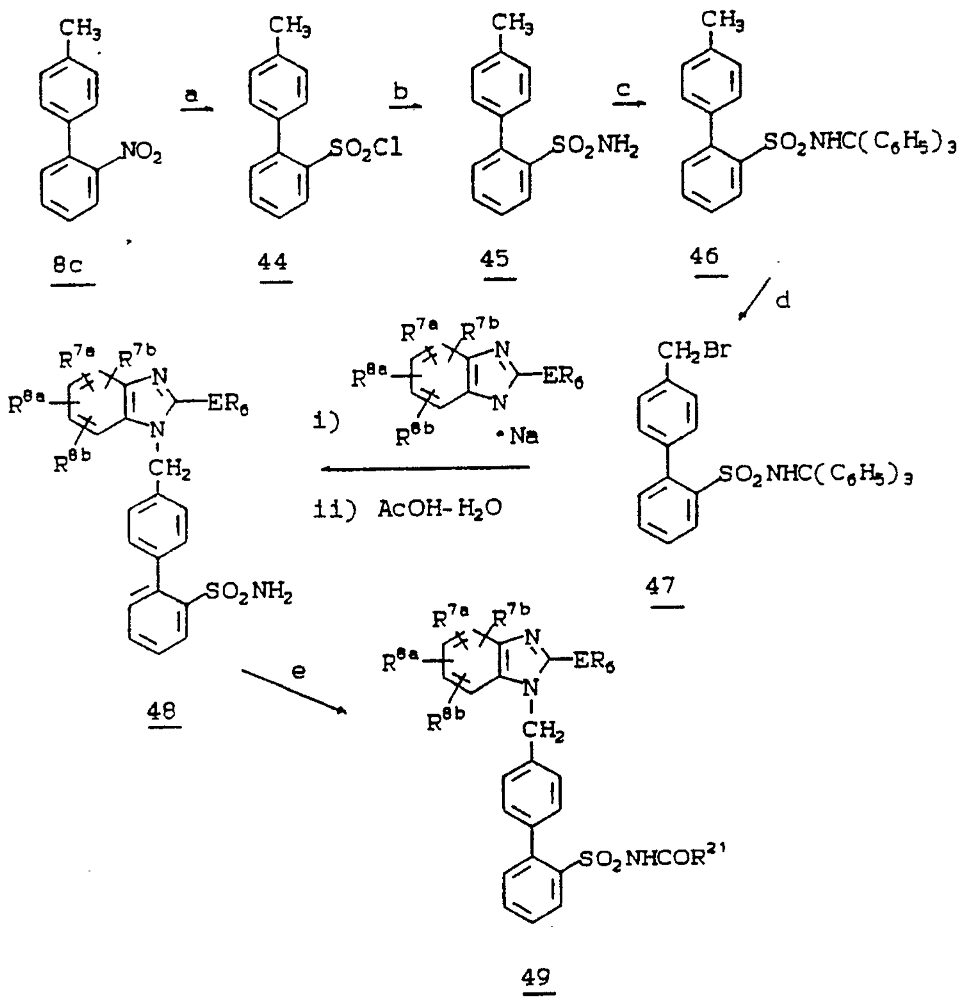


8c 44 45 46 47 48 49

a) i) $H_2$/Pd-C, ii) $NaNO_2$-HCl, iii) $SO_2$, AcOH, $CuCl_2$

b) $NH_3$ or $(NH_4)_2CO_3$

c) $(C_6H_5)_3CCl$, $Et_3N$, $CHCl_3$, reflux

d) N-Bromosuccinimide, AIBN, $CCl_4$, reflux

e) $R^{21}COCl$ or $R^{21}CO$-Im or other acylating agents.

sulfur-dioxide in the presence of a copper(II) salt to form the corresponding arylsulfonylchloride 44 [H. Meerwein, G. Dittmar, R. Gollner, K. Hafner, F. Mensch and O. Steifort - Chem. Ber., 90, 841 (1957); A. J. Prinsen and H. Cerfontain, Recueil, 84, 24 (1965); E. E. Gilbert, Synthesis, 3 (1969) and references cited therein]. The sulfonyl chloride can be reacted with ammonia in aqueous solution or in an inert organic solvent [F. H. Bergheim and W. Baker, J. Amer. Chem. Soc., 66, (1944), 1459], or with dry powdered ammonium carbonate, [E. H. Huntress and J. S. Autenrieth, J. Amer. Chem. Soc., 63, (1941), 3446; E. H. Huntress and F. H. Carten, J. Amer. Chem. Soc., 62, (1940), 511] to form the sulfonamide 45. The benzylbromide 47 may be prepared from the sulfonamide 45 as outlined in Scheme 8, and then can be reacted with an alkali metal salt of an appropriate heterocyclic compound to form the key sulfonamide 48.

The sulfonamide 48 may be also prepared from the aromatic sulfonyl chloride 53, which may be prepared from the aryl amine 52 as outlined in Scheme 9. The acylation of 48 with appropriate acyl chlorides (or acyl-imidazoles or other acylating agents) may produce the desired acylsulfonamides 49.

The compounds bearing $R^1$ as $-SO_2NHR^{21}$ (where $R^{21}$ is heteroaryl) may be prepared by reacting the aromatic sulfonyl chloride 53 with appropriate heteroaryl amines as outlined in Scheme 9. The sulfonyl chloride 53 may be the

SCHEME 9

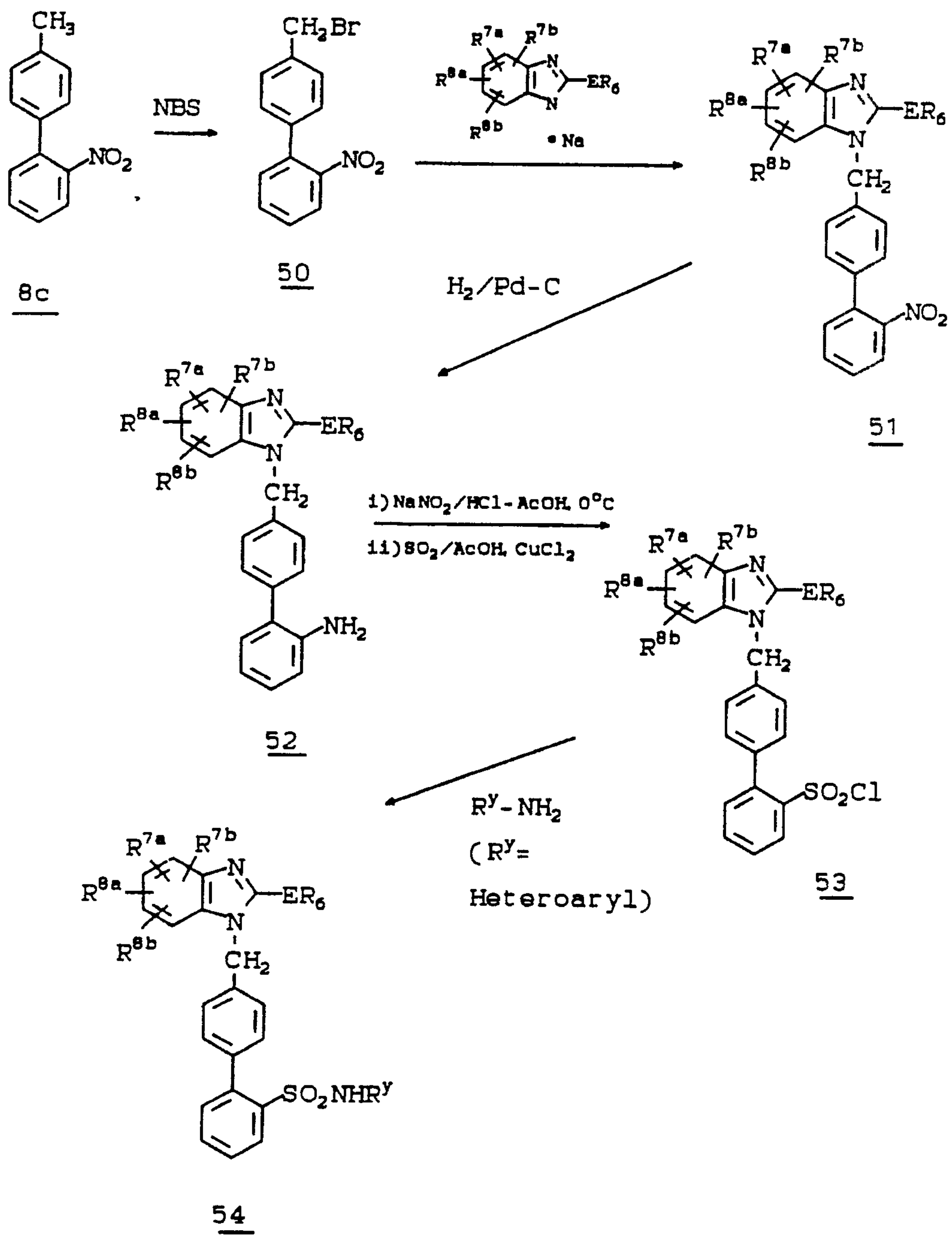


8c

50

51

52

53

54

SCHEME 10

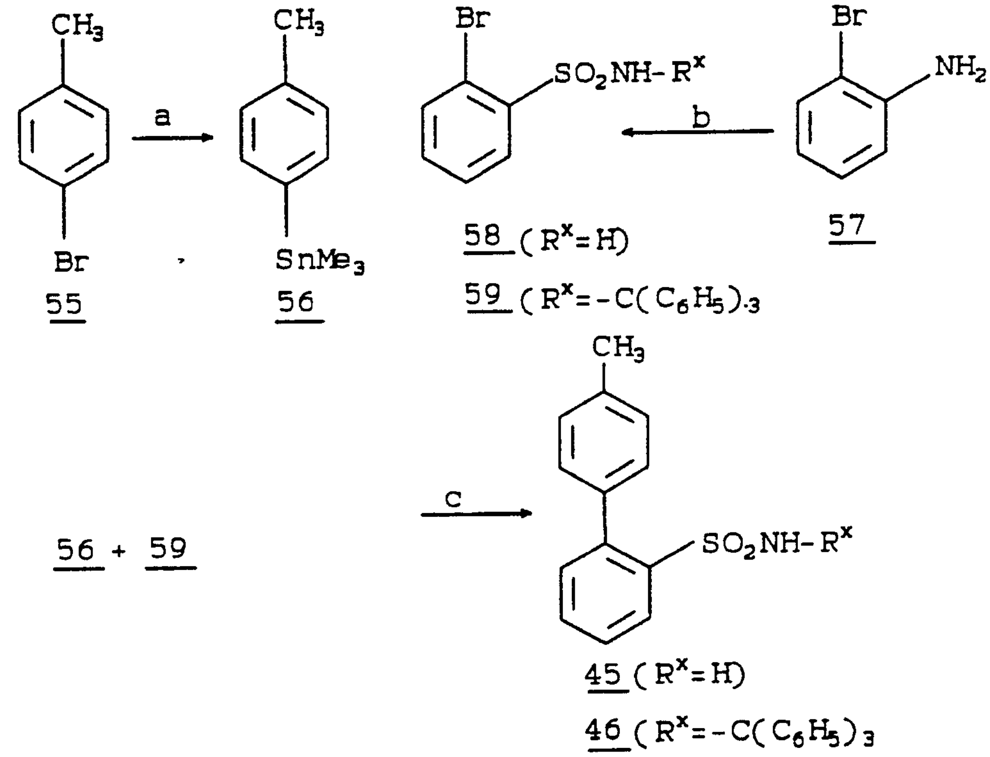


55 56 58 ($R^x$=H) 59 ($R^x$=-C($C_6H_5$)$_3$) 57

56 + 59

45 ($R^x$=H)
46 ($R^x$=-C($C_6H_5$)$_3$

a. (i) t-BuLi/ether, -78°C (ii) $Me_3SnCl$
b. (i) $NaNO_2$/HCl (ii) $SO_2$, $CuCl_2$
c. $Pd(PPh_3)_4$, Toluene or $(PPh_3)_2PdCl_2$, THF.

preferred intermediate for the synthesis of this class of compounds. The aromatic sulfonyl chlorides may also be prepared by reacting the sodium salt of aromatic sulfonic acids with $PCl_5$ or $POCl_3$ [C. M. Suter, The Organic Chemistry of Sulfur, John Wiley & Sons, 459, (1944)]. The aromatic sulfonic acid precursors may be prepared by chlorosulfonation of the aromatic ring with chlorosulfonic acid [E. H. Huntress and F. H. Carten, J Amer. Chem. Soc., 62, 511 (1940)].

The biaryl sulfonamides 45 and 46 (described in Scheme 8) can be prepared alternatively using palladium(O) catalyzed cross-coupling reactions of appropriate aryl-organotin precursors [J. K. Stille, Pure Appl. Chem., 57, 1771 (1985); T. R. Baiely, Tetra Let., 27, 4407 (1986); D. A. Widdowson and Y. Z. Zhang, Tetrahedron, 42, 2111 (1986)], as outlined in Scheme 10. The organotin compound 56 [S. M. Moerlein, J. Organometallic Chem., 319, 29 (1987)], obtained from the aromatic precursor 55, may be coupled with aryl sulfonamide 59 using $Pd(PPh_3)_4$ or $(PPh_3)_2PdCl_2$ as catalysts to give biaryl sulfonamide 46. Similarly, the benzyl bromide 47 may be alternatively prepared from the appropriate organotin precursor 62 using the Pd-(O) catalyzed cross-coupling reaction as outlined in Scheme 11.

## SCHEME 11

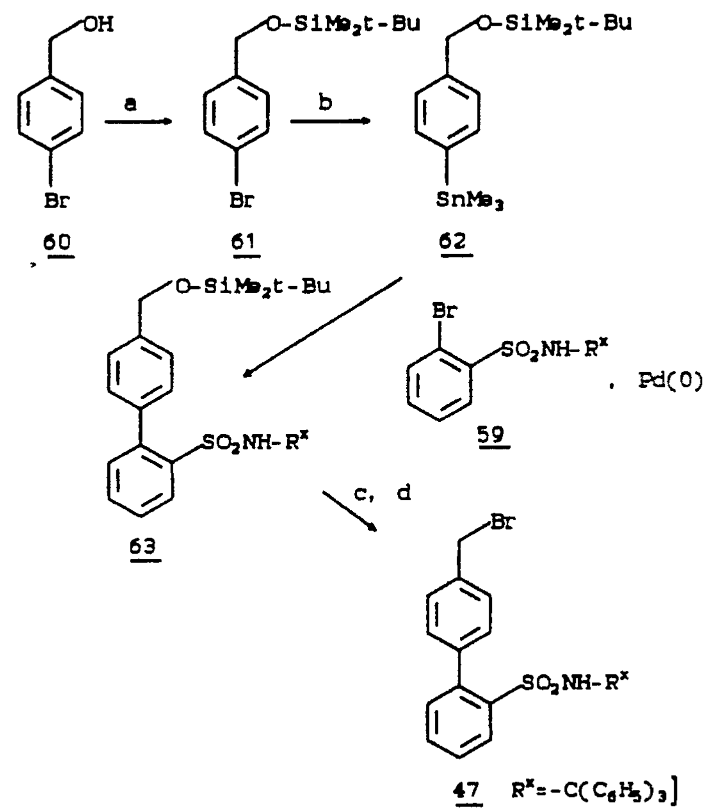


60 61 62 59 63 47

a. t-Bu $Me_2Si-Cl$/Imidazole, DMF

b. t-BuLi, -78°C, $Me_3SnCl$

c. Tetrabutylammonium fluoride

d. $CBr_4/Ph_3P$.

## SCHEME 12

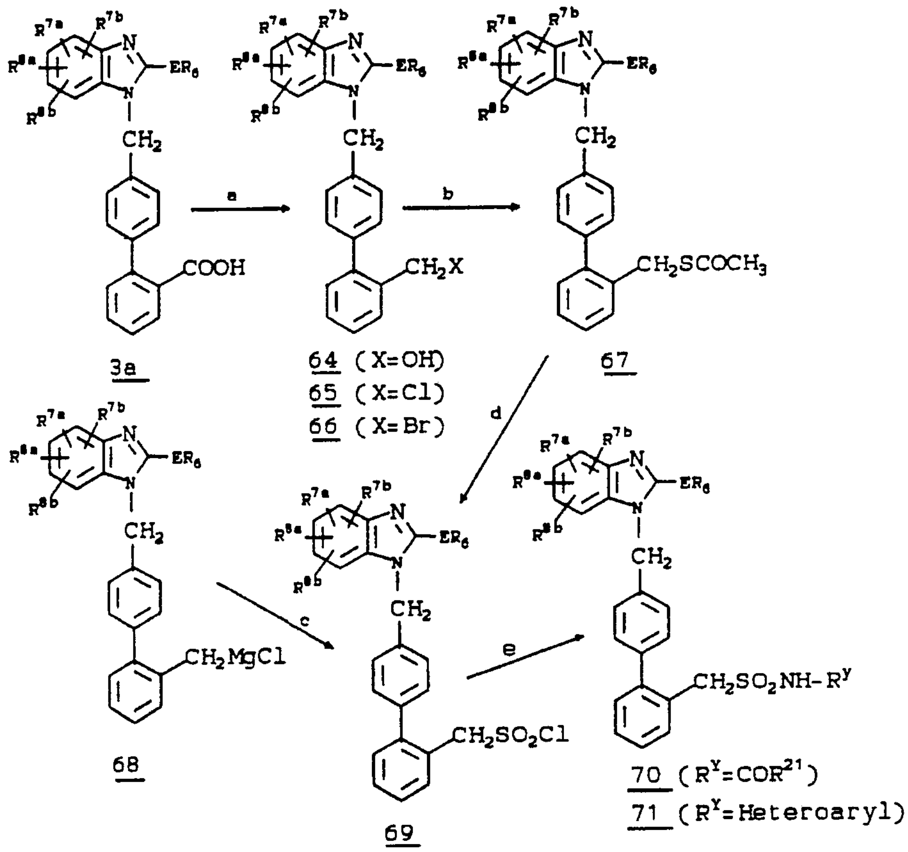


3a

64 (X=OH)
65 (X=Cl)
66 (X=Br)

67

68

69

70 ($R^Y$=$COR^{21}$)
71 ($R^Y$=Heteroaryl)

a. i) $EtOCOCl/Et_3N$, THF, 0°C ii) $NaBH_4$ iii) $CCl_4$ or $CBr_4/PPh_3$
b. AcSK
c. $SO_2Cl_2$
d. $Cl_2$, AcOH, $H_2O$ or, i) $SO_2Cl_2$ ii) oxidation
e. $R^yNH_2$ or, i) $NH_3$ ii) Acylation.

The compounds bearing $R^1$ = $-CH_2SO_2NHCOR^{21}$ and $-CH_2SO_2NHR^{21}$ may be prepared as outlined in Scheme 12. The key precursor aryl-methanesulfonyl chloride 69 may be prepared either from the reaction of aryl-methylmagnesium chloride 68, obtained from the corresponding benzyl chloride 65, with sulfurylchloride [S. N. Bhattacharya, C. Eaborn and D. P. M. Walton, J. Chem Soc. C, 1265 (1968)], or by oxidation of the aryl-methylthioacetate 67 (prepared from the benzyl bromide 66) with chlorine in presence of trace amount of water [Bagnay and Dransch, Chem. Ber., 93, 784 (1960)]. Alternatively, the aryl-methylthioacetate 67 can be oxidized with sulfuryl chloride in presence of acetic anhydride to form aryl-methylsulfinyl chloride [S. Thea and G. Cevasco, Tetra. Lett., 28, 5193 (1987)], which can be further oxidized with appropriate oxidizing agents to give the sulfonyl chloride 69. The compounds 70 and 71 can be obtained by reacting the sulfonyl chloride 69 with appropriate amines.

Compounds 74 where $R^1$ = $-NHSO_2NHR^{2'}$ may be prepared by the reaction of appropriate primary amines with the sulfamide 73 [S. D. McDermott and W. J. Spillane, Synthesis, 192 (1983)], as described in Scheme 13. The compound 73 may be obtained from the corresponding N-t-butylsulfamide 72 after treatment with anhydrous trifluoroacetic acid [J. D. Catt and W. L. Matier, J. Org. Chem., 39, 566 (1974)], which may be prepared by the reaction of the aromatic amine 52 with t-butylsulfamoyl chloride [W. L.

Matier, W. T. Comer and D. Deitchman, J. Med. Chem., 15, 538 (1972)].

SCHEME 13

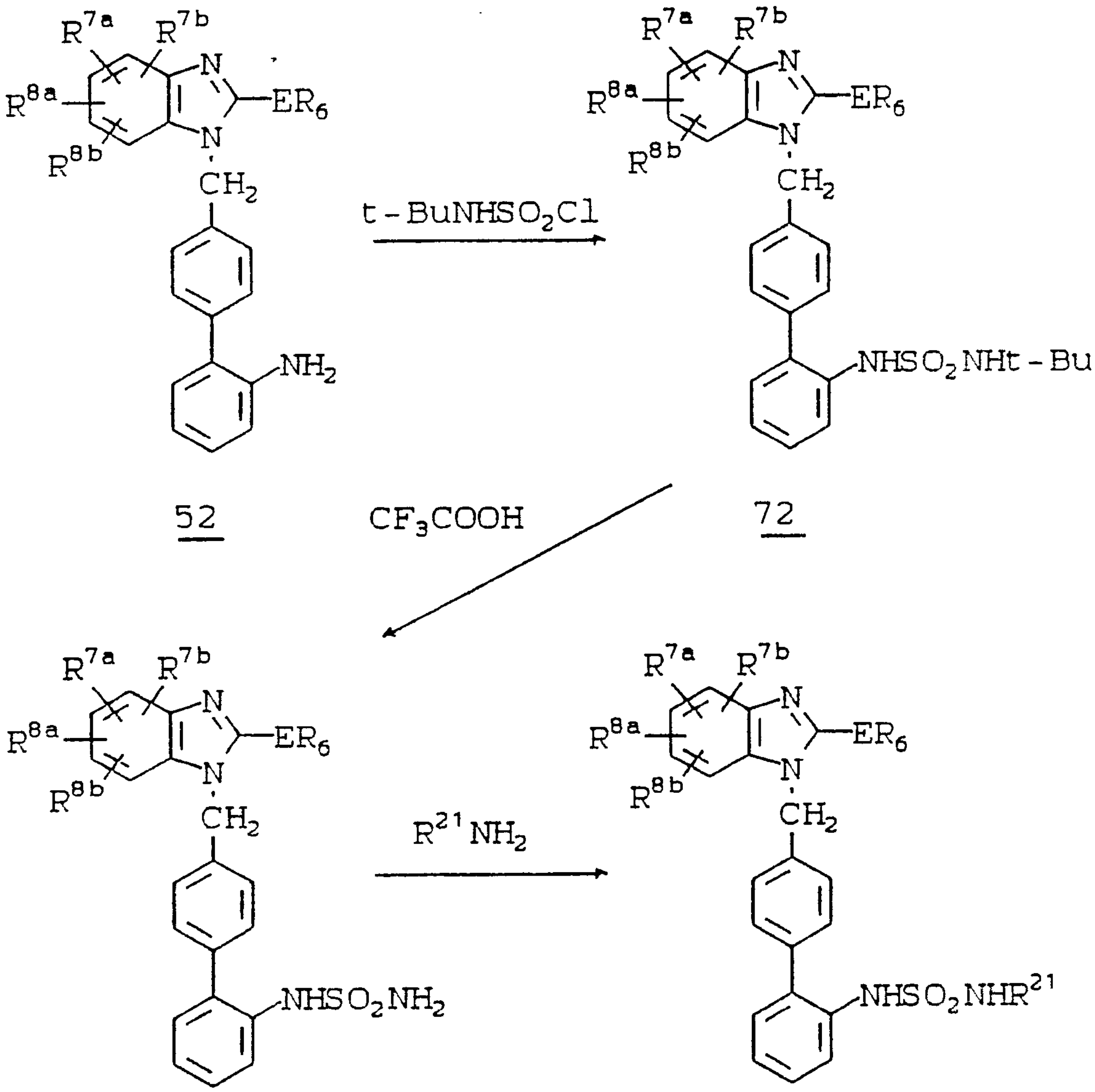


52 72 73 74

The compounds of this invention form salts with various inorganic and organic acids and bases which are also within the scope of the invention. Such salts include ammonium salts, alkai metal salts like sodium and potassium salts, alkaline earth metal salts like the calcium and magnesium salts, salts with organic bases; e.g, dicyclohexylamine salts, N-methyl-D-glucamine, salts with aminoa acids like arginine, lysine, and the like. Also, salts with organic and inorganic acids may be prepared; e.g., HCl, HBr, $H_2SO_4$, $H_3PO_4$, methane-sulfonic, toluensulfonic, maleic, fumaric, camphorsulfonic. The non-toxic, physiologically, acceptable salts are preferred, although other salts are also useful; e.g., in isolating or purifying the product.

The salts can be formed by conventional means such as by reacting the free acid or free base forms of

the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Angiotensin II (AII) is a powerful arterial vasoconstrictor, and it exerts its action by interacting with specific receptors present on cell membranes. The compounds described in the present invention act as competitive antagonists of AII at the receptors. In order to identify AII antagonists and determine their efficacy in vitro, the following two ligand-receptor binding assays were established.

Receptor binding assay using rabbit aortae membrane preparation:

Three frozen rabbit aortae (obtained from Pel-Freeze Biologicals) were suspended in 5mM Tris-0.25M Sucrose, pH 7.4 buffer (50 ml) homogenized, and then centifuged. The mixture was filtered through a cheesecloth and the supernatant was centrifuged for 30 minutes at 20,000 rpm at 4° C. The pellet thus obtained was resuspended in 30 ml of 50mM Tris-5 mM $MgCl_2$ buffer containing 0.2% Bovine Serum Albumin and 0.2 mg/ml Bacitration and the suspension was used for 100 assay tubes. Samples tested for screening were done in duplicate. To the membrane preparation (0.25 ml) there was added $^{125}$I-Sar$^1$Ile$^8$-angiotensin II [obtained from New England Nuclear] (10μl; 20,000 cpm) with or without the test sample and the mixture was incubated at 37° C for 90 minutes. The mixture was then diluted with ice-cold 50mM Tris-0.9% NaCl, pH 7.4 (4ml) and filtered through a glass fiber filter (GF/B Whatman 2.4" diameter). The filter was soaked in scintillation cocktail (10 ml) and counted for radioactivity using Packard 2660 Tricarb liquid scintillation counter. The inhibitory concentration ($IC_{50}$) of potential AII antagonist which gives 50% displacement of the total specifically bound $^{125}$I-Sar$^1$Ile$^8$-angiotensin II was presented as a measure of the efficacy of such compounds as AII antagonists.

Receptor assay using Bovine adrenal cortex preparation

Bovine adrenal cortex was selected as the source of AII receptor. Weighed tissue (0.1 g is needed for 100 assay tubes) was suspended in Tris.HCl (50mM), pH 7.7 buffer and homogenized. The homogenate was centrifuged at 20,000 rpm for 15 minutes. Supernatant was discarded and pellets resuspended in buffer [$Na_2HPO_4$ (10mM)-NaCl (120mM)-disodium EDTA (5mM) containing phenylmethane sulfonyl fluoride (PMSF)(0.1mM)]. (For screening of compounds, generally duplicates of tubes are used). To the membrane preparation (0.5 ml) there was added 3H-angiotensin II (50mM) (10μl) with or without the test sample and the mixture was incubated at 37° C for 1 hour. The mixture was then diluted with Tris buffer (4ml) and filtered through a glass fiber filter (GF/B Whatman 2.4" diameter). The filter was soaked in scintillation cocktail (10ml) and counted for radioactivity using Packard 2660 Tricarb liquid scintillation counter. The inhibitory concentration ($IC_{50}$) of potential AII antagonist which gives 50% displacement of the total specifically bound $^3$H-angiotensin II was presented as a measure of the efficacy of such compounds as AII antagonists.

The potential antihypertensive effects of the compounds described in the present invention may be evaluated using the methodology described below:
Male Charles River Sprague-Dawley rats (300-375 gm) were anesthetized with methohexital (Brevital; 50 mg/kg i.p.) and the trachea was cannulated with PE 205 tubing. A stainless steel pithing rod (1.5 mm thick, 150 mm long) was inserted into the orbit of the right eye and down the spinal column. The rats were immediately placed on a Harvard Rodent Ventilator (rate - 60 strokes per minute, volumn -1.1 cc per 100 grams body weight). The right carotid artery was ligated, both left and right vagal nerves were cut, and the left carotid artery was cannulated with PE 50 tubing for drug administration, and body temperature was maintained at 37° C by a thermistatically controlled heating pad which received input from a rectal temperature probe. Atropine (1 mg/kg i.v.) was then administered, and 15 minutes later propranolol (1 mg/kg i.v.). Thirty minutes later angiotensin II or other agonists were administered intravenously at 30-minute intervals and the increase in the diastolic blood pressure was recorded before and after drug or vehicle administration.

Using the methodology described above, representative compounds of the invention were evaluated and all were found to exhibit an activity of at least $IC_{50}$<50μM thereby demonstrating and confirming the utility of the compounds of the invention as effective AII antagonists.

Thus, the compounds of the invention are useful in treating hypertension. They are also of value in the management of acute and chronic congestive heart failure, in the treatment of secondary hyperaldosteronism, primary and secondary pulmonary hyperaldosteronism, primary and secondary pulmonary hypertension, renal failure such as diabetic nephropathy, glomerulonephritis, scleroderma, and the like, renal vascular hypertension, left ventricular dysfuction, diabetic retinopathy and in the management of vascular disorders such as migraine or Raynaud's disease. The application of the compounds of this invention for these and similar disorders will be apparent to those skilled in the art.

The compounds of this invention are also useful to treat elevated intraocular pressure and can be administered to patients in need of such treatment with typical pharmaceutical formulations such as tablets, capsules, injectables and the like as well as topical ocular formulations in the from of solutions ointments, inserts, gels, and the like. Pharmaceutical formulations prepared to treat intraocular pressure would typically contain about 0.1% to 15% by weight, preferably 0.5% to 2% by weight, of a compound of this invention.

In the management of hypertension and the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 1 to 1000 mg. per patient per day which can be administered in single or multiple doses. Perferably, the dosage range will be about 2.5 to 250 mg. per patient per day; more preferably about 2.5 to 75 mg. per patient per day.

The compounds of this invention can also be administered in combination with other antihypertensives and or diuretics and or angiotensin converting enzyme inhibitors and or calcium channel blockers. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methyclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, and the like, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the angiotensin II antagonists of this invention effective clinically in the 2.5-250 milligrams per day range can be effectively combined at levels at the 0.5-250 milligrams per day range with the following compounds at the indicated per day dose range: hydrochlorothiazide (15-200 mg) chlorothiazide (125-2000 mg), ethacrynic acid (15-200 mg), amiloride (5-20 mg), furosemide (5-80 mg), propranolol (20-480 mg), timolol maleate (5-60 mg.), methyldopa (65-2000 mg), felodipine (5-60 mg), nifedipine (5-60 mg), and nitrendipine (5-60 mg). In addition, triple drug combinations of hydrochlorothiazide (15-200 mg) plus amiloride (5-20 mg) plus angiotensin II antagonist of this invention (3-200 mg) or hydrochlorothiazide (15-200 mg) plus timolol maleate (5-60) plus an angiotensin II antagonist of this invention (0.5-250 mg) or hydrochlorothiazide (15-200 mg) and nifedipine (5-60 mg) plus an angiotensin II antagonist of this invention (0.5-250 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can he formulated into pharmaceutical compositions as discussed below.

About 1 to 100 mg. of compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a

lubricant such as magnesium stearate; a sweetening agent such as sucrose, lactose or saccharin; a flavoring agent such as peppermint, oil of wintergreen or cherry. When the dosage unitform is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occuring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

## DETAILED DESCRIPTION OF THE INVENTION

The following examples are set forth to illustrate the invention and should not be construed as limiting the invention recited in the appended claims.

### Example 1

### 2-Propyl-1-(2′-carboxybiphen-4-yl)methylbenzimidazole

#### Step A: 2-Propylbenzimidazole

To a solution of o-phenylenediamine [obtained from the corresponding dihyrochloride (1.0 g, 5.52 mmol)] in methanol (30 ml) was added $Cu(OAc)_2$ (3.64 g, 18.2 mmol) as an aqueous solution (60 ml). After stirring for 10-15 minutes at room temperature, butyraldehyde (0.80 g, 11.1 mmol) was added and the mixture was heated at 100° C for 4 hours. Additional butyraldehyde (0.2 g) was added, and the mixture was stirred at room temperature overnight. The reaction was filtered and the residue was dissolved in methanol (40 ml). To this solution $H_2S$ gas was bubbled for about 10 minutes followed by a stream of nitrogen. CuS was filtered-off, and the filtrate was evaporated in vacuo. The crude product was then purified by flash chromatography using ethyl acetate/hexane (3:1) to give the desired product (0.14 g, 16%). NMR($CD_3OD$): $\delta$ 1.0 (t, J=7Hz, 3H), 1.88 (m, 2H), 2.86 (t, J=7Hz, 2H), 7.2 (m, 2H), 7.5 (m, 2H); FAB-MS: m/e 161 (M+H).

#### Step B: 2-t-Butoxycarbonyl-4′-methylbiphenyl

To a solution of p-bromotoluene (30 g) in dry ether (150 ml) at -78° C, a solution of t-BuLi in pentane (1.7M) (210 ml) was added slowly over a period of 1 hour and 30 minutes using a dropping funnel. The bath was then removed and the mixture was stirred at room temperature for an additional 2 hours. The contents of the flask were then added slowly (using a cannula) at room temperature to a premixed solution of $ZnCl_2$ in ether (1M) (180 ml) and dry THF (360 ml). The mixture was stirred for 2 hours at that temperature and the slurry was added (using a cannula) to a solution of 2-t-butoxycarbonyl iodobenzene (35.6 g,) and $NiCl_2(Ph_3P)_2$ (2.1 g) in dry THF (360 ml). The mixture, after stirring at room temperature overnight (18 hours), was poured slowly with stirring into ice-cold 0.5N HCl (1500 ml). The organic layer was separated and the aqueous phase was extracted with ether (3 X 300 ml). The combined organic layer was washed with water, brine and then dried over $MgSO_4$. Removal of the solvent gave the crude product as an oil (32 g). The material was purified on a silica-gel flash column using ethylacetate-hexane (1:12) to give the titled compound as an oil (24 g, 76%). NMR(CDCl3): $\delta$ 1.24 (s, 9H) 2.42 (s, 3H), 7.2-7.8 (m, 8H); FAB-MS: m/e 269 (M+H).

#### Step C: 2-Propyl-1-(2′-t-butoxycarbonylbiphen-4-yl-methylbenzimidazole

To a suspension of Nak (0.013 g) in dry DMF (3ml), 2-propyl benzimidazole (0.052 g) was added at room temperature and the mixture was stirred at 40° C for 30 minutes to give a clear solution. To the solution was added t-butyl 4′-bromomethyl biphenyl-2-carboxylate (0.113 g) [prepared according to the procedure described in Reaction Scheme 2 above], and the mixture was stirred at room temperature for 3 hours. The mixture was poured into ice-water (50 ml) and extracted with ethyl acetate (3 x 20 ml). The combined organic phase was washed with brine, then dried ($MgSO_4$) and evaporated. The crude product was then purified by flash-chromatography on silica-gel using ethyl acetate-hexane (5:30). Yield 0.1 g (72%) (amorphous solid). NMR($CDCl_3$): δ 1.0 (t, J=7Hz, 3H), 1.26 (s, 9H), 1.88 (m, 2H), 2.86 (t, J=7Hz, 2H), 5.4 (s, 2H), 7.05-7.50 (m, 10H), 7.8 (m, 2H); FAB-MS: m/e 427 (M+H).

Step D: 2-Propyl-1-(2′-carboxybiphen-4-yl)methylbenzimidazole

The t-butyl ester derivative (0.060 g, 0.14 mmol), described in Step C, was dissolved in $CH_2Cl_2$-TFA (1:1) (2 ml) [containing anisole (0.05 ml)], and the mixture was stirred for 1 hour. The residue obtained, after removal of the solvent in vacuo, was triturated with dry ether and filtered to give the desired acid as cream colored solid (0.045 g, 86 %). NMR($CD_3OD$): δ 1.0 (t, J=7Hz, 3H), 1.88 (m, 2H), 2.86 (t, J=7Hz, 2H), 5.25 (s, 2H), 7.2-7.9 (m, 12H); FAB-MS: m e 371 (M+H).

## Example 2

2-Propyl-1-(4-(2′-carboxybenzoyl)phenyl )methylbenzimidazole

Step A: 2-Propyl-1-(4-(2′-t-butoxycarbonylbenzoyl)phenyl)methylbenzimidazole

To a suspension of NaH (0.013 mg) in dry DMF (3 ml), 2-propyl benzimidazole (0.052 g, 0.325 mmol) was added, and the mixture was stirred at room temperature for 30 minutes to give a clear solution. To the solution was added t-butyl-2-[4′-(bromomethyl)benzoyl]benzoate (0.125 g, 0.33 mmol) [prepared according to the procedure described in European Patent Application 0,253,310]. The mixture, after stirring at room temperature for 3 hours, was poured into ice-water (50 ml) and extracted with ethyl acetate (3 x 20 ml). The combined organic phase was washed with brine, then dried ($MgSO_4$), and evaporated. The crude product was then purified by flash chromatography on silica-gel using ethyl acetate-hexane (1:6). Yield 0.12 g (81%, as amorphous solid). NMR($CDCl_3$): δ 1.0 (t, J=7Hz, 3H), 1.26 (s, 9H), 1.88 (m, 2H), 2.86 (t, J=7Hz, 2H), 5.4 (s, 2H), 7.05-7.8 (m, 12H); FAB-MS: m e 455 (M+H).

Step B: 2-Propyl-1-(4-(2′-carboxybenzoyl)phenyl)methylbenzimidazole

The t-butyl ester derivative (0.050 g), described in Step A, was dissolved in $CH_2Cl_2$-TFA (1:1) (2 ml) [containing anisole (0.05 ml)], and the mixture was stirred for 2 hours. The residue obtained, after removal of the solvent in vacuo, was triturated with dry ether and filtered to give the desired acid as white solid (0.037 g, 84%). NMR($CD_3OD$): δ 1.0 (t, J=7Hz, 3H), 1.88 (m, 2H), 2.86 (t, J=7Hz, 2H), 5.4 (s, 2H), 7.05-7.50 (m, 10H), 7.8 (m, 2H); FAB-MS: 399 (M+H).

## Example 3

2-Butyl-1-(2′-carboxybiphen-4-yl)methylbenzimidazole

Step A: 2-Butylbenzimidazole

To a solution of o-phenylenediamine (0.171 g, 1.58 mmol) in absolute ethanol (8 ml), ethyl valeroylimidate (0.313 g, 1.9 mmol) was added, and the mixture was refluxed overnight. The reaction was cooled to room temperature and concentrated in vacuo. The residue was partitioned between ethyl acetate and saturated $NaHCO_3$. The organic phase was separated and the aqueous layer was extracted with ethyl acetate (3 x 20 ml). The organic layers were combined and washed with brine , dried ($MgSO_4$), and then concentrated in vacuo. The crude product, thus obtained, was purified by medium pressure liquid chromatography (MPLC) on silica-gel using ethyl acetate-hexane (1:1) to give cream colored crystalline solid (0.19 g, 69%). NMR ($CDCl_3$): $\delta$ 0.92 (t, J=7Hz, 3H), 1.42 (m, 2H), 1.86 (m, 2H), 2.95 (t, J=7Hz, 2H), 7.22 (m, 2H), 7.58 (m, 2H); FAB-MS: m/e 174 (M+H).

Step B: t-Butyl-2-Butyl-1-(2′-t-butoxycarbonylbiphen-4-yl)methylbenzimidazole

To a suspension of NaH (0.024 g, 0.5 mmol, as 50% oil suspension) in dry DMF (3 ml), 2-butyl benzimidazole (0.087 g, 0.5 mmol) was added at room temperature and stirred at 40° C for 30 minutes to give a clear solution. To the solution was added t-butyl 4′-bromomethylbiphenyl-2-carboxylate (0.19 g, 0.55 mmol). The mixture was stirred at room temperature for 3 hours, and then poured into ice-water (50 ml) and extracted with ethylacetate (3 x 30 ml). The combined organic phase was washed with brine, then dried ($MgSO_4$) and evaporated. The crude product was then purified by flash chromatography on silica-gel using ethyl acetate-hexane (1:2). Yield 0.15 g (68%) (amorphous solid). NMR($CDCl_3$): $\delta$ 0.95 (t, J=7Hz, 3H), 1.24 (s, 9H), 1.45 (m, 2H), 1.86 (m, 2H), 2.95 (t, J=7Hz, 2H), 5.38 (s, 2H), 7.05-7.8 (m, 12H); FAB-MS; m/e 441 (M+H)

Step C: 2-Butyl-1-(2′-carboxybiphen-4-yl)methylbenzimidazole

The t-butyl ester derivative (0.060 g, 0.136 mmol), described in Step B, was dissolved in $CH_2Cl_2$-TFA (1:1) (2 ml) [containing anisole (0.05 ml)], and the mixture was stirred for 1 hour. The residue obtained, after removal of the solvent in vacuo, was triturated with dry ether and filtered to give the desired acid as white solid (0.043 g, 82%). NMR($CDCl_3$): $\delta$ 0.95 (t, J=7Hz, 3H), 1.45 (m, 2H), 1.86 (m, 2H), 3.15 (t, J=7Hz, 2H), 5.52 (s, 2H), 7.1-7.95 (m, 12H); FAB-MS: m/e 385 (M+H)

## Example 4

### 2-Butyl-4-methyl-1-(2′-carboxybiphen-4-yl)methylbenzimidazole

Step A: 4-Methyl-2-butylbenzimidazole

A mixture of 3-methylphenylene-1,2-diamine (0.491 g, 4.02 mmols), valeric acid (0.459 ml, 4.23 mmols) and polyphosphoric acid (3 ml) was heated at 60° C for 2 days. The reaction was cooled, diluted with water (10 ml) and adjusted to pH 10 with conc. $NH_4OH$. The mixture was extracted with $CHCl_3$ (3 x 30 ml) and the combined oganic phase was washed with water and brine, dried ($MgSO_4$), and then concentrated in vacuo. The crude product, thus obtained, was purified by medium pressure liquid chromatography (MPLC) on silica-gel using ethyl acetate-hexane (1:1) to give cream colored solid (0.756 g). NMR($CDCl_3$): $\delta$ 0.95 (t, J=7Hz, 3H), 1.42 (m, 2H), 1.85 (m, 2H), 2.55 (s, 3H), 3.0(t, J=7Hz, 2H), 7.0-7.2 (m, 2H), 7.35 (d, J=100z, 1H); FAB-MS: m/e 189 (M+H).

Step B: 2-Butyl-4-methyl-1-(2′-t-butoxycarbonylbiphen-4-yl)methylbenzimidazole

2-Butyl-4-methyl benzimidazole (0.094 g, 0.5 mmol) was alkylated with t-butyl 4′-bromomethyl biphenyl-2-carboxylate (0.19 g, 0.55 mmol), according to the procedure described in Example 1. The crude product on TLC showed to be a mixture of two regioisomers. The material was purified by MPLC on silica-gel using ethyl acetate-hexane (1:1). The higher $R_f$ material (0.090 g, 40%; as a glass-like solid) was the desired

product. NMR($CDCl_3$): δ 0.95 (t, J=7Hz, 3H), 1.24 (s, 9H), 1.42 (m, 28), 1.85 (m, 2H), 2.70 (s, 3H), 2.92 (t, J=7Hz, 2H), 5.4 (s, 2H), 7.0-7.8 (m, 11H); FAB-MS: m/e 455 (M+H). The lower $R_f$ isomer was the undesired regioisomer (0.020 g, 9%). NMR($CDCl_3$): δ 0.95 (t, J=7Hz, 3H), 1.24 (s, 9H), 1.42 (m, 2H), 1.85 (m, 2H), 2.55 (s, 3H), 2.82 (t, J=7Hz, 2H), 5.62 (s, 2H), 6.95-7.8 (m, 11H).

Step C: 2-Butyl-4-methyl-1-(2′-carboxybiphen-4-yl)methylbenzimidazole

The higher $R_f$ t-butyl ester derivative (0.078 g) described in Step B, was deprotected according to the procedure described in Step D of Example 1. The crude product obtained after work-up was purified on a LH-20 column using MeOH to give the titled product as a glass like solid (0.051 g). NMR($CDCl_3$): δ 0.90 (t, J=7Hz, 3H), 1.42 (m, 2H), 1.75 (m, 2H), 2.68 (s, 3H), 3.22 (t, J=7Hz, 2H), 5.48 (s, 2H), 7.0-7.85 (m, 11H); FAB-MS:m/e 399 (M+H).

## Example 5

2-Butyl-7-methyl-1-(2′-carboxybiphen-4-yl)methylbenzimidazole

The lower $R_f$ regioisomer (0.010 g) described in Step B of Example 4, was deprotected, according to the procedure described in Step D of Example 1, to give the titled compound (0.006 g). NMR($CDCl_3$): δ 0.95 (t, J=7Hz, 3H), 1.42 (m, 2H), 1.75 (m, 2H), 2.55 (s, 3H), 3.10 (t, J=7Hz, 2H), 5.68 (s, 2H), 6.95-7.9 (m, 11H); FAB-MS: m/e 399 (M+H).

## Example 6

2-Butyl-4,5-dimethyl-1-(2′-carboxybiphen-4-yl)methylbenzimidazole

Step A: 2-Butyl-4,5-dimethylbenzimidazole

The titled compound was prepared from 3,4-dimethyl-phenylene-1,2-diamine (0.272 g, 2 mmols) according to the procedure described in Step A of Example 1. The crude product was purified by flash column chromatography on silica-gel using ethyl acetate-hexane (1:2). Yield 0.375 g (93%), as a cream colored solid. NMR($CDCl_3$): δ 0.95 (t, J=7Hz, 3H), 1.42 (m, 2H), 1.82 (m, 2H), 2.38(s, 6H), 2.90 (t, J=7Hz, 2H), 7.05 (d, 2H) ; FAB-MS: m/e 203 (M+H).

Step B: 2-Butyl-4,5-dimethyl-1-(2′-t-butoxycarbonylbiphen-4-yl)methylbenzimidazole

2-butyl-3,4-dimethyl benzimidazole (0.101 g, 0.5 mmol) was alkylated with t-butyl 4′-bromomethyl biphenyl-2-carboxylate (0.19 g, 0.55 mmol), according to the procedure described in Step C of Example 1. The crude product on TLC showed only one major (>90%) regioisomer. The material was purified by flash-chromatography on silica-gel using ethyl acetate-hexane (1:3). Yield 0.13 g (as a foam). NMR($CDCl_3$): δ 0:94 (t, J=7Hz, 3H), 1.24 (s, 9H), 1.44 (m, 2H), 1.82 (m, 2H), 2.39 (s, 3H), 2.62 (s, 3H), 2.89 (t, J=7Hz, 2H), 5.36 (s, 2H), 6.95-7.8 (m, 10H) FAB-MS: m/e 469 (M+H)

Step C: 2-Butyl-4,5-dimethyl-1-(2′-carboxybiphen4-yl)methylbenzimidazole

The t-butyl ester derivative (0.10 g) described in Step B, was deprotected according to the procedure described in Step D of Example 1. The crude product obtained after work-up was crystallized from methanol-ether to give the pure titled compound as white solid (0.072 g). NMR($CD_3OD$): δ 0.97 (t, J=7Nz,

. 3H), 1.46 (m, 2H), 1.75 (m, 2H), 2.47 (s, 3H), 2.58 (s, 3H), 3.24 (t, J=7Hz, 2H), 5.76 (s, 2H), 7.2-7.85 (m, 10H); FAB-MS: m/e 413 (M+H). Analysis calcutated for $C_{27}N_{28}N_2O_2$ ^ $CF_3COOH$ ^ $0.7H_2O$: Ψ, 64.72%; H, 5.69%; N, 5.21%. Found: C, 64.45%; H, 5.60%; N, 5.00%

## Example 7

### 2-Butyl-5.6-dimethyl-1-(2′-carboxybiphen-4-yl)methylbenzimidazole

#### Step A: 2-Butyl-5,6-dimethylbenzimidazole

The titled compound was prepared from 4,5-dimethyl-phenylene-1,2-diamine (0.272 g, 2 mmols) according to the procedure described in Example 1. The crude product was purified by crystallization from ethyl acetate-hexane (1:1). Yield 0.357 g (88%), as a cream colored solid. NMR($CDCl_3$): δ 0.95 (t, J=7Hz, 3H), 1.42 (m, 2H), 1.82 (m, 2H), 2.35 (s, 6H) 2.90 (t, J=7Hz, 2H), 7.32 (br s, 2H) ; FAB-MS: m/e 203 (M+H).

#### Step B: 2-Butyl-5,6-dimethyl-1-(2′-t-butoxycarbonylbiphen-4-yl)methylbenzimidazole

To a suspension of NaH (0.028 g, 0.7 mmol, as 50% oil suspension) in dry DMF (3m1), 2-butyl-5,6-dimethylbenzimidazole (0.101 g, 0.5 mmol) was added at room temperature and the mixture stirred at 40° C for 30 min to give a clear solution. To the solution was added t-butyl 4′-bromomethylbiphenyl-2-carboxylate (0.19 g, 0.55 mmol). The mixture was stirred at room temperature for 3 hours at 40° C, cooled and then poured into ice-water (50 ml) and extracted with ethyl acetate (3 x 30 ml). The combined organic phase was washed with brine, then dried ($MgSO_4$) and evaporated. The crude product was then purified by flash chromatography on silica-gel using ethyl acetate-hexane (1:3). Yield 0.157 g (68%) (amorphous solid). NMR($CDCl_3$): δ 0.95 (t, J=7Hz, 3H), 1.24 (s, 9H), 1.42 (m, 2H), 1.82 (m, 2H), 2.32 (s, 3H), 2.38 (s, 3H), 2.84 (t, J=7Hz, 2H), 5.36 (s, 2H), 7.0-7.8 (m, 10H) ; FAB-MS; m/e 469 (M+H).

#### Step C: 2-Butyl-5,6-dimethyl-1-(2′-carboxybiphen-4-yl)methylbenzimidazole

The t-butyl ester derivative (0.065 g, 0.14 mmol), described in Step B, was dissolved in $CH_2Cl_2$-TFA (1:1) (2 ml) [containing anisole (0.05 ml)], and the mixture was stirred for 3 hours. The residue obtained, after removal of the solvent in vacuo, was triturated with dry ether and filtered to give the desired acid as white solid (0.043 g, 82%). The material was recrystallized from methanol-ether. Yield, 0.34 g (46%); NMR-($CD_3OD$): δ 0.964 (t, J=7Hz, 3H), 1.45 (m, 2H), 1.78 (m, 2H), 2.44 (s, 3H), 2.46 (s, 3H), 3.20 (t, J=7Hz, 2H), 5.75 (s, 2H), 7.25-7.85 (m, 10H) ; FAB-MS: m/e 413 (M+H). Analysis calcutated for $C_{27}H_{28}N_2O_2$ ^ $CF_3COOH$ ^ $0.7H_2O$: C, 64.72%; H, 5.69%; N, 5.21%. Found: C, 64.45%; H, 5.60%; N, 5.00%

## Example 8

### 2-Butyl-5,6-dichloro-1-(2′-carboxyphen-4-yl)methylbenzimidazole

#### Step A: 2-Butyl-5,6-dichlorobenzimidazole

The titled compound was prepared from 4,5-dichloro-phenylene-1,2-diamine (0.708 g, 4 mmol) and ethyl valeryl imidate (0.99 g, 6 mmols), according to the procedure described in Example 1. The crude product was purified by flash chromatography using ethyl acetate-hexane (1:3). The product, thus obtained, was then crystallized from ethyl acetate/hexane (1:1). Yield 0.55 g (57%), as colorless crystalline solid. mp,

NMR($CDCl_3$): δ 0.95 (t, J=7Hz, 3H), 1.42 (m, 2H), 1.82 (m, 2H), 2.90 (t, J=7Hz, 2H), 7.62 (s, 2H); FAB-MS: m/e 243 (M+H).

Step B: 2-Butyl-5,6-dichloro-1-(2′-t-butoxycarbonylbiphen-4-yl)methylbenzimidazole

2-Butyl-5,6-dichloro benzimidazole (0.121 g, 0.5 mmol) was alkylated with t-butyl 4′-bromomethyl biphenyl-2-carboxylate (0.19 g, 0.55 mmol), according to the procedure described in Example 1. The crude product was then purified by flash-chromatography on silica-gel using ethyl acetate-hexane (1:3). The material was recrystallized from ethyl acetate hexane (1:1). Yield 0.074 g (30%). NMR ($CDCl_3$): δ 0.95 (t, J=7Hz, 3H), 1.24 (s, 9H), 1.45 (m, 2H), 1.86 (m, 2H), 2.87 (t, J=7Hz, 2H), 5.36(s, 2H), 7.034 (d, J=10Hz, 2H), 7.26-7.83 (m, 8H); FAB-MS; m e 509, 511 (M+H).

Step C: 2-Butyl-5,6-dichloro-1-(2′-carboxybiphen-4-yl)methylbenzimidazole

The t-butyl ester derivative (0.050 g, 0.10 mmol), described in Step B, was deprotected according to the procedure described in Step D of Example 1. The crude product obtained after work-up was crystallized from methanol-ether to give the pure titled compound as white solid (0.024 g, 53%). NMR($CD_3OD$): δ 0.95 (t, J=7Hz, 3H). 1.43 (m, 2H), 1.75 (m, 2H), 2.92 (t, J=7Hz, 2H), 5.53 (s, 2H), 7.1-7.8 (m, 10H) ; EAB-MS: m/e 453, 455 (M+H). Analysis calculated for $C_{25}H_{22}N_2O_2Cl_2$ ^ $CF_3COOH$ ^ $0.5H_2O$: C, 64.96; H, 5.01; N6.06. Found: C, 64.75; H, 4.87; N, 5.85.

## Example 9

2-Butyl-1-(2′-carboxybiphen-4-yl)methyl-1H-naphth[2,3-d]imidazole

Step A: 2-Butyl-1H-naphth[2,3-d]imidazole

A mixture of 2,3-naphthylenediamine (0.32 g, 2 mmols) and ethyl valeroimidate (0.495 g, 3 mmols) in absolute ethanol (16 ml) was refluxed for 24 hours. The reaction was cooled to room temperature and filtered to remove unreacted starting material. The filtrate was concentrated in vacuo and the residue was treated with saturated aqueous $NaNCO_3$, as described in Example 1. The crude product was purified by flash chromatography on silica-gel using ethyl acetate/hexane (2:3) to give 0.55 g of the product, which was recrystallized from ethyl acetate-hexane (1:1). Yield 0.32 g (70%), as a cream colored solid. NMR($CDCl_3$): δ 0.964 (t, J=7Hz, 3H), 1.47 (m, 2H), 1.92 (m, 2H), 3.0 (t, J=7Hz, 2H), 7.40 (m, 2H), 7.96 (m, 4H); FAB-MS: m/e 225 (M+H).

Step B: 2-Butyl-1-(2′-t-butoxycarbonylbiphen-4-yl)methyl-1H-naphth[2,3-d]imidazole

2-Butyl-1H-naphth[2,3-d]imidazole (0.112 g, 0.5 mmol) was alkylated with t-butyl 4′-bromomethyl biphenyl-2-carboxylate (0.19 g, 0.55 mmol), according to the procedure described in Example 1. The crude product was then purified by flash chromatography on silica-gel using ethyl acetate-hexane (1:3). Yield 0.17g(70%). NMR($CDCl_3$): δ 0.98 (t, J=7Hz, 3H), 1.2(s, 9H), 1.48 (m, 2H), 1.94 (m, 2H), 2.95 (t, J=7Hz, 2H), 5.49 (s, 2H), 7.1-8.2 (m, 10H); FAB-MS; m e 490.62 (M+H).

Step C: 2-Butyl-1-(2′-carboxybiphen-4-yl)methyl1H-naphth[2,3-d]imidazole

The t-butyl ester derivative (0.1 g, 0.20 mmol), described in Step B, was deprotected according to the procedure described in Step D of Example 1. The crude product obtained after work-up was crystallized from methanol-ether to give the pure titled compound as white solid (0.070 g, 65%). NMR($CD_3OD$): δ 1:0 (m, 3H), 1.52 (m, 2H), 1.87 (m, 2H), 3.30 (m, 2H), 5.85 (s, 2H), 7.30-7.62 (m, 9H), 7.83 (d, J=10.4Hz, 1H),

8.11 (m, 2H), 8.29 (s, 2H); FAB-MS: m/e 435(M+H). Analysis calculated for $C_{29}H_{26}N_2O_2Cl_2$ ^ $CF_3COOH$ ^ $0.5H_2O$: C, 66.77; H, 5.06; N, 5.02. Found: C, 67.09; H, 5.18; N, 4.75.

Example 10

2-Butyl-5-carbomethoxy-1-(2′-carboxybiphen-4-yl)methylbenzimidazole

Step A: 2-Butyl-5-carbomethoxybenzimidazole

The titled compound was prepared from methyl 3,4-diaminobenzoate (0.333 g, 2 mmols) according to the procedure described in Example 1. The crude product was purified by crystallization from ethyl acetate-hexane (1:1). Yield 0.327 g (70%), as a cream colored solid. NMR($CDCl_3$): δ 0.95 (t, J=7Hz, 3H), 1.42 (m, 2H), 1.85 (m, 2H), 2.92 (t, J=7Hz, 2H), 3.93 (s, 3H), 7.26 (s, 1H), 7.95 (dd, $J_1$ =2Hz and $J_2$ =11Hz, 2H); FAB-MS: m/e 233(M+H)

Step B: 2-Butyl-5-carbomethoxy-1-(2′-t-butoxycarbonylbiphen-4-yl)methylbenzimidazole

2-Butyl-5-carbomethoxy benzimidazole (0.116 g, 0.5 mmol) was alkylated with t-butyl 4′-bromomethyl biphenyl-2-carboxylate (0.19 g, 0.55 mmol), according to the procedure described in Example 1. The crude product was then purified by flash chromatography on silica-gel using ethyl acetate-hexane (1:1). The product was obtained as a mixture of N-1 and N-3 alkylated regioisomers in 3:2 ratio. Seperation of isomers was not possible, and the mixture was carried to the next step. Yield 0.17 g (70%). NMR($CDCl_3$): δ 0.96 (t, J=7Hz, 3H), 1.2 and 1.23 (two singlets for two isomers, 9H), 1.46 (m, 2H), 1.88 (m, 2H), 2.90 (t, J=7Hz, 2H), 3.91 and 3.94 (two singlets for two isomers, 3H), 5.42 (s, 2H; for isomer 1) and 5.46 (s, 2H for isomer B), 7.05-8.03 (m, 11 H); FAB-MS: m/e 499(M+H).

Step C: 2-Butyl-5-carbomethoxy-1-(2′-carboxybiphen-4-yl)methylbenzimidazole

The t-butyl ester derivative (0.08 g) described in Step B, was deprotected according to the procedure described in Step D of Example 1. The crude product obtained after work-up was crystallized from methanol-ether to give the pure titled compound as white solid (0.044g). NMR($CD_3OD$): δ 0.90 (t, J=7Hz, 3H), 1.40 (m, 2H), 1.74 (m, 2H), 2.98 (m, 2H), 3.86 and 3.89 (two singlets for two isomers, 3H), 5.58 (s, 2H; for isomer 1) and 5.62 (s, 2H for isomer B), 7.05-8.3 (m, 11H); FAB-MS: m/e 443 (M+H). Analysis calculated for $C_{27}H_{26}N_2O_4$ ^ $1.3H_2O$: C, 69.59; H, 6.18; N, 6.01. Found: C, 69.31; H, 5.71; N, 5.81.

Example 11

2-Butyl-1-(2′-(tetrazol-5-yl)biphen-4-yl)methylbenzimidazole

Step A: 2-cyano-4′-methylbiphenyl

To a solution of p-bromotoluene (30 g) in dry ether (150 ml) at -78° C, a solution of t-BuLi in pentane (1.7M) (210 ml) was added slowly over a period of 1 hour. and 30 minutes, using a dropping funnel. The bath was then removed and the mixture was stirred at room temperature for an additional 2 hours. The contents of the flask were then added slowly (using a cannula) at room temperature to a premixed soluiton of $ZnCl_2$ in ether (1M) (180 ml) and dry THF (360 ml). The mixture was stirred for 2 hours at that temperature and the the slurry was added (using a cannula) to a solution of 2-bromobenzonitrile (21.3 g) and $NiCl_2(Ph_3P)_2$ (2.1 g) in dry THF (300 ml). The mixture, after stirring at room temperature overnight (18

h), was poured slowly with stirring into ice-cold 1N HCl (1500 ml). The organic layer was separated and the aqueous phase was extracted with ether (3 X 300ml). The combined organic layer was washed with water, brine and then dried over $MgSO_4$. Removal of the solvent gave the crude product as a semisolid mass (34 g). The material was purified on a silica-gel flash column using ethyl acetate hexane (1:12) to give the desired nitrile as a low-melting solid (28 g, 88%). NMR($CDCl_3$): δ 2.42 (S,3H), 7.2-7.8 (m,8H); FAB-MS: m e 194 (M+H).

Step B: Trimethylstannyl azide

To a concentrated solution of $NaN_3$ (40 g) in water (100 ml), a solution of trimethyltin chloride (20 g) in dioxane (10 ml) was added in three portions under vigorous stirring. A precipitate formed instantneously. The mixture, after stirring overnight at room temperature, was filtered. The residue was washed with water, and dried under suction and then in vacuo over $P_2O_5$. Yield 18.7 g (81%), mp 132-136 °C.

Step C: 2-Butyl-1-(2′-(N-triphenylmethyltetrazol-5-yl)-biphen-4-yl)methylbenzimidazole

To a suspension of NaH (0.024 g, 0.5 mmol, as 50% oil suspension) in dry DMF (3 ml), 2-butyl benzimidazole ( 0.087 g, 0.5 mmol) was added at room temperature and stirred at 40 °C for 30 minutes to give a clear solution. To the solution was added N-triphenylmethyl-5-[2-(4′-bromomethylbiphen-4-yl)-]tetrazole (0.30 g, 0.55 mmol) [prepared from 2-cyano-4′-methylbiphenyl using trimethylstannyl azide procedure for tetrazole formation described in European Patent 0291,969]. The mixture, after stirring at room temperature for 4 hours, was poured into ice-water (50 ml) and extracted with ethylacetate (3 x 30 ml). The combined organic phase was washed with brine, then dried ($MgSO_4$) and evaporated. The crude product was then purified by MPLC on silica-gel using ethylacetate-hexane (1:1). Yield 0.20 g (62 %) (amorphous solid). NMR ($CDCl_3$): δ 0.92 (t, J=7Hz, 3H), 1.4 (m, 2H), 1.82 (m, 2H), 2.78 (t, J=7Hz, 2H), 5.22 (s, 2H), 6.8-8.0 (m, 27H); FAB-MS: m e 651(M+H).

Step D: 2-Butyl-1-(2′-(tetrazol-5-yl)-biphen-4-yl)methylbenzimidazole

The protected derivative (0.313 g) obtained from Step C was suspended in 50% aqueous AcOH (6 ml), and the mixture was heated at 60 °C for 2 hours and then left at room temperature for 24 hours. The solvent was removed in vacuo, and the residue was purified on LH-20 using methanol to give the desired product as glass-like solid (0.19 g) after evaporation of the solvent. NMR ($CDCl_3$): δ 0.92 (t, J=7Hz, 3H), 1.4 (m, 2H), 1.82 (m, 2H), 2.78 (t, J=7Hz, 2H), 5.22 (s, 2H), 6.8-8.2 (m, 12H); FAB-MS: m e 409 (M+H).

Example 12

2-Butyl-4-methyl-1-(2′-(tetrazol-5-yl)-biphen-4-yl)methylbenzimidazole

The titled compound was prepared from 2-butyl-4-methyl benzimidazole according to the procedures described in Steps C and D of Example 11. The deprotected material was purified by flash-chromatography on silica-gel using $CHCl_3$-MeOH-$NH_4OH$ (40:10:1) solvent system. NMR ($CDCl_3$): δ 0.92 (t, J = 7H), 3H), 1.4 (m, 2H), 1.82 (m, 2H), 2.58 (s, 3H), 2.78 (t, J= 7Hz, 2H), 5.22 (s, 2H), 6.8-8.2 (m, 11H); FAB-MS: m.e 423 (M+H).

Example 13

2-Propyl-4-methyl-1-2′-(tetrazol-5-yl)biphen-4-yl)methylbenzimidazole

2-Propyl-4-methyl benzimidazole was alkylated with N-triphenylmethyl-5-[2-(4′-bromomethylbiphen-4-yl)]tetrazole according to the procedure described in Step C of Example 11. The trityl protecting group was removed according to the method described in Step D of Example 11 and the deprotected material was purified by flash-chromatography on silica-gel using $CHCl_3$-MeOH-$NH_4OH$ (40:10:1) solvent system. NMR ($CDCl_3$): δ 1.0 (t, J = 7Hz, 3H), 1.88 (m, 2H), 2.62 (s, 3H), 2.86 (t, J = 7Hz, 2H), 5.22 (s, 2H), 6.8-8.2 (m, 11H); FAB-MS: m/e 409 (M + H).

## Example 14

### 2-Butyl-5-carbomethoxy-(2′-(tetrazol-5-yl)biphen-4-yl)methylbenzimidazole

The titled compound was prepared from 2-butyl-5-carbomethoxybenzimidazole (described in Step A of Example 10) according to the procedures described in Steps C and D of Example 11. The isomers 1 and 2 formed during the alkylation step were carried to the deprotection step directly. The deprotected material was purified by flash-chromatography on silica-gel using $CHCl_3$-MeOH-$NH_4OH$ (40:10:1) solvent system. NMR ($CD_3OD$): δ 0.92 (t, J = 7Hz, 3H), 1.4 (m, 2H), 1.77 (m, 2H), 2.98 (t, J = $7H_z$, 2H), 3.86 and 3.89 (two singlets for two isomers, 3H) 5.58 (singlet for isomer 1, 2H), 5.66 (s, isomer 2, 2H), 6.8-8.2 (m, 11H); FAB-MS: m/e 467(M + H).

## Example 15

### 2-Butyl-7-hydroxymethyl-1-(2′-(tetrazol-5-yl)biphen-4-yl)methylbenzimidazole

The mixture of isomers containing the trityl protecting group obtained in Example 14 was treated with diisobutylaluminium hydride (6 equivalents) in THF at -78° C to produce a mixture containing the titled compound and the corresponding regioisomer. The titled compound was separated by flash chromatography using ethylacetate-hexane (3:2). Elution of the column with ethylacetate gave the 5-hydroxymethyl isomer (lower $R_f$ material). The protecting group was removed according to the procedure described in Step D of Example 11 and the material was purified on silica-gel using $CHCl_3$-MeOH-$NH_4OH$ (40:10:1) solvent system. NMR($CD_3OD$): Satisfactory for the 7-hydroxymethyl isomer. FAB-MS: m.e 439(M + H).

## Example 16

### 2-Butyl-5-hydroxymethyl-1-(2′-(tetrazol-5-yl)biphen-4-yl)methylbenzimidazole

The lower $R_f$ material obtained in Example 15 was found to be the desired titled compound. The protecting group was removed according to the procedure described in Step D of Example 11 and the material was purified on silica-gel using $CHCl_3$-MeOH-$NH_4ON$ (40:10:1) solvent system. NMR($CD_3OD$): Satisfactory for the desired product. FAB-MS: m/e 439(M + H).

## Example 17

### 2-Butyl-1-(2′-(N-phenylsulfonyl)carboxamidobiphen-4-yl)methylbenzimidazole

To a suspension of 2-butyl-1-(2′-carboxybiphen-4-yl)methylbenzimidazole (0.0675 g, 0.1755 mmol) (obtained from Step C of Example 3) was added thionyl chloride (0.12 ml, 1.76 mmol) and the mixture was

refluxed for 3 hours. The reaction was cooled and evaporated in vacuo to give the corresponding acid chloride, which was dried in vacuo for 2 hours. In a separate flask, benzenesulfonamide (0.03 g) was added to a supension of NaH (0.01 g) in dry DMF (1 ml), and the mixture was stirred at 40° C for 30 minutes and then cooled to room temperature. To this was added a solution of the acid chloride in DMF (1 ml), and the mixture was stirred at room temperature for 24 hours. The reaction was poured into ice-water (50 ml), acidified with 5% citric acid and extracted with ethylacetate (3 x 20 ml). The combined organic sphase was washed with brine, dried over $MgSO_4$ and then evaporated in vacuo giving the crude product as a foam. The material was then purified by flash-chromatography on silica-gel using chloroform-methanol-$NH_4OH$ (40:10:1) to give the titled product as a glass-like solid (0.066 g, 71%). NMR ($CDCl_3$): δ 0.95 (t, J=7Hz, 3H), 1.45 (m, 2H), 1.88 (m, 2H), 2.85 (t, J=7Hz, 2H), 5.38 (s, 2H), 6.9-7.8 (m, 18H). FAB-MS: m/e 524 (M+H).

## Example 18

### 4-Methyl-2-propyl-1-(2′-(tetrazol-5-yl)biphen-4-yl)methylbenzimidazole

#### Step A: 4-Methyl-2-propylbenzimidazole

To a solution of 2,3-diaminotoluene (0.574 g, 4.69 mmol) in absolute ethanol (18 ml) was added ethyl butyrylimidate hydrochloride (0.923 g, 5.64 mmol) (prepared by the reaction of butyronitrile with HCl gas in absolute ethanol), and the mixture was refluxed for 17 hours. At the end of this period, the solvent was removed in vacuo, and the residue was partitioned between chloroform and saturated $NaHCO_3$. The organic phase was washed with brine and dried over $MgSO_4$. Removal of the solvent gave the crude product as a foam, which was then purified by flash-chromatography on silica-gel using ethylacetate-hexane (1:1) to give the pure tilted compound as an amorphous solid (0.43 g, 56%). NMR ($CDCl_3$): δ 0.98 (t, J=7Hz, 3H) 1.82 (m, 2H), 2.56 (s, 3H), 2.91 (m, 2H), 6.98-7.42 (m, 4H). FAB-MS: m/e 175 (M+H).

#### Step B: 4-Methyl-2-propyl-1-(2′-(N-triphenylmethyltetrazol-5-yl)biphen-4-yl)methylbenzimidazole

To a suspension of NaH (0.047 g, 1.18 mmol) in dry DMF (10 ml) was added 4-Methyl-2-propyl benzimidazole (0.205 g, 1.18 mmol), and the mixture was stirred at 40° C for 30 minutes. The solution was cooled and N-triphenylmethyl-5-[2-(4′-bromomethylbiphen-4-yl)tetrazole (0.676 g, 1.24 mmol) was added. The mixture, after stirring for 18 hours at room temperature, was poured into ice-water (100 ml) and extracted with ethyl acetate (3 x 30 ml). The combined organic phase was washed with brine and dried over $MgSO_4$. Removal of the solvent in vacuo gave the crude product as a foam, which was then purified by flash-chromatography on silica-gel using ethyl acetate/hexane (1:2). Yield: 0.49 g (65%, as a foam). NMR ($CDCl_3$): δ 0.97 (t, J=7.0Hz, 3H), 1.8 (m, 2H), 2.72 (s, 3H), 2.78 (t, J=7.0Hz, 2H), 5.20 (s, 2H), 6.75-7.5 (m, 25H), 7.93 (m, 1H). FAB-MS: m/e 651 (M+H).

#### Step C: 4-Methyl-2-propyl-1-(2′-(tetrazol-5-yl)biphen-4-yl)methylbenzimidazole

The protected compound (0.256 g, 0.393 mmol), obtained from Step B, was suspended in 50% aqueous acetic acid (10 ml) and refluxed for 3 hours. The reaction was cooled and concentrated in vacuo. The residue obtained was triturated with dry ether and filtered, washed with dry ether and dried in vacuo over $P_2O_5$ overnight. Yield: 0.126 g (81%, amorphous solid). NMR ($CDCl_3$): δ 0.9 (t, J=7.0Hz, 3H), 1.68 (m, 2H), 2.10 (s, 3H), 2.16 (t, J=7.0Hz, 2H), 522 (s, 2H), 6.72 (d, J=8Hz, 2H), 6.87-7.10 (m, 6H), 7.3 (m, 1H), 7.55 (m, 2H), 7.88 (m, 1H). FAB-MS: m/e 409 (M+H) and 817 (2M+H).

## Example 19

4-Methyl-2-propyl-1-(2'-carboxybiphen-4-yl)methylbenzimidazole

The titled compound was prepared from 4-Methyl-2-propylbenzimidazole (from Step A of Example 18) according to the procedure described in Steps B and C of Example 5. NMR ($CD_3OD$): δ 1.0 (t, J=7Hz, 3H), 1.8 (m, 2H), 2.62 (s, 3H), 3.0 (t, J=7.0Hz, 2H), 5.57 (s, 2H), 7.1-7.7 (m, 11H), FAB-MS: m/e 385 (M+H)>.

## Example 20

Typical Pharmaceutical Compositions Containing a Compound of the Invention

| A: Dry Filled Capsules Containing 50 mg of Active Ingredient Per Capsule | |
|---|---|
| Ingredient | Amount per capsule (mg) |
| 2-butyl-1-(2'-(tetrazol-5-yl)biphen-4-yl)methylbenzimidazole | 50 |
| Lactose | 149 |
| Magnesium stearate | 1 |
| Capsule (size No. 1) | 200 |

The 2-butyl-1-(2'-(tetrazol-5-yl)biphen-4-yl)methylbenzimidazole can be reduced to a No. 60 powder and the lactose and magnesium stearate can then be passed through a No. 60 blotting cloth onto the powder. The combined ingredients can then be mixed for about 10 minutes and filled into a No. 1 dry gelatin capsule.

B: Tablet

A typical tablet would contain 2-butyl-1-(2'-(tetrazol-5-yl)biphen-4-yl)methylbenzimidazole (25 mg), pregelatinized starch USP (82 mg), microcrystaline cellulose (82 mg) and magnesium stearate (1 mg).

C: Combination Tablet

A typical combination tablet would contain, for example, a diuretic such as hydrochlorothiazide and consist of 2-butyl-1-(2'-(tetrazol-5-yl)biphen-4-yl)methylbenzimidazole (7.5 mg), hydrochlorothiazide (50 mg) pregelatinized starch USP (82 mg), microcrystalline cellulose (82 mg) and magnesium stearate (1 mg).

D: Suppository

Typical suppository formulations for rectal administration can contain 2-butyl-1-(2'-(tetrazol-5-yl)biphen-4-yl)methylbenzimidazole (1-25 mg), butylated hydroxyanisol (0.08-1.0 mg), disodium calcium edetate (0.25-0.5 mg), and polyethylene glycol (775-1600 mg). Other suppository formulations can be made by substituting, for example, butylated hydroxytoluene (0.04-0.08 mg) for the disodium calcium edetate and a hydrogenated vegetable oil (675-1400 mg) such as Suppocire L, Wecobee FS, Wecobee M, Witepsols, and the like, for the polyethylene glycol. Further, these suppository formulations can also include another active ingredient such as another antihypertensive and/or a diuretic and/or an angiotensin converting enzyme and/or a calcium channel blocker in pharmaceutically effective amounts as described, for example, in C above.

E: Injection

A typical injectible formulation would contain 2-butyl-1-(2'-(tetrazol-5-yl)biphen-4-yl)methylbenzimidazole (5.42 mg), sodium phosphate dibasic anhydrous (11.4 mg) benzylalcohol (0.01 ml) and water for injection (1.0 ml). Such an injectible formulation can also include a pharmaceutically effective amount of another active ingredient such as another antihypertensive and/or a diuretic and/or an angiotensin converting enzyme inhibitor and/or a calcium channel blocker.

Utilizing the procedure and methods described in the foregoing Examples and in the Reaction Schemes, additional compounds of the invention can be prepared as shown in Table I below:

## TABLE I

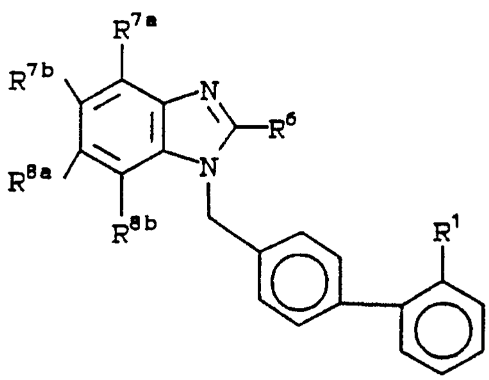

(Ia)

| $R^1$ | $R^6$ | $R^{7a}$ | $R^{7b}$ | $R^{8a}$ | $R^{8b}$ |
|---|---|---|---|---|---|
| COOH | Propyl | $CH_3$ | F | H | H |
| 1H-Tetrazol-5-yl | Propyl | $CH_3$ | F | H | H |
| 1H-Tetrazol-5-yl | Butyl | $CH_3$ | F | H | H |
| COOH | Propyl | H | H | F | $CH_3$ |
| 1H-Tetrazol-5-yl | Propyl | H | H | F | $CH_3$ |
| 1H-Tetrazol-5-yl | Propyl | $CH_3$ | H | $CH_2OH$ | H |
| 1H-Tetrazol-5-yl | Butyl | $CH_3$ | H | $CH_2OH$ | H |

| R1 | $R^6$ | $R^{7a}$ | $R^{7b}$ | $R^{8a}$ | $R^{8b}$ |
|---|---|---|---|---|---|
| 1H-Tetrazol-5-yl | $S-CH_3$ | $CH_3$ | H | $CH_2OH$ | H |
| COOH | Propyl | $CH_3$ | F | $CH_2OH$ | H |
| 1H-Tetrazol-5-yl | Propyl | $CH_3$ | F | $CH_2OH$ | H |
| 1H-Tetrazol-5-yl | Propyl | $CH_3$ | $CH_2OH$ | F | H |
| 1H-Tetrazol-5-yl | Butyl | $CH_3$ | $CH_2OH$ | F | H |
| 1H-Tetrazol-5-yl | Propyl | $CH_3$ | F | $CH_2OCONH_2$ | H |
| 1H-Tetrazol-5-yl | Butyl | $CH_3$ | F | $CH_2OCONH_2$ | H |
| $-NHSO_2CF_3$ | Propyl | $CH_3$ | F | $CH_2OCONH_2$ | H |
| 1H-Tetrazol-5-yl | $CH_2SCH_3$ | $CH_3$ | F | $CH_2OCONH_2$ | H |
| 1H-Tetrazol-5-yl | Propyl | H | $NHCOOC_2H_5$ | H | H |
| COOH | Propyl | H | H | $NHCOOC_2H_5$ | H |
| 1H-Tetrazol-5-yl | Propyl | H | H | $NHCOOC_2H_5$ | H |
| $-NHSO_2CF_3$ | Propyl | H | H | $NHCOOC_2H_5$ | H |
| 1H-Tetrazol-5-yl | Propyl | $CH_3$ | F | $NHCOOC_2H_5$ | H |
| 1H-Tetrazol-5-yl | Butyl | $CH_3$ | F | $NHCOOC_2H_5$ | H |
| 1H-Tetrazol-5-yl | Propyl | $CH_3$ | F | $NHSO_2CH_3$ | H |
| 1H-Tetrazol-5-yl | Propyl | $CH_3$ | H | $SO_2NHCH_3$ | H |
| 1H-Tetrazol-5-yl | Propyl | H | F | $CH_2OH$ | H |
| 1H-Tetrazol-5-yl | Propyl | $CH_3$ | $NHSO_2CH_3$ | H | H |
| 1H-Tetrazol-5-yl | Propyl | H | $NHSO_2CH_3$ | H | H |
| 1H-Tetrazol-5-yl | Propyl | $C_2H_5$ | $NHSO_2CH_3$ | H | H |
| 1H-Tetrazol-5-yl | Propyl | $CH_3$ | $N(CH_3)_2$ | H | H |
| 1H-Tetrazol-5-yl | Butyl | $CH_3$ | $N(CH_3)_2$ | H | H |
| 1H-Tetrazol-5-yl | Propyl | $CH_3$ | H | $N(CH_3)_2$ | H |

## Claims

1. A compound having the formula:

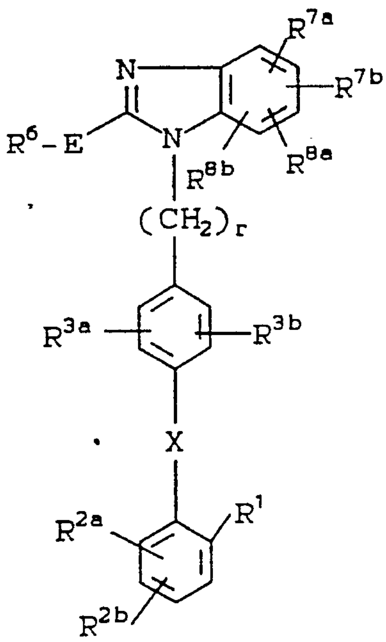

(I)

R' is

(a) $-CO_2R^4$,
(b) $-SO_3R^5$,
(c) $-NHSO_2CF_3$,
(d) $-PO(OR^5)_2$,
(e) $-SO_2-NH-R^9$,
(f) $-CONHOR^5$,
(g) $-SO_2NH$-heteroaryl,
(h) $-CH_2SO_2NH$-heteroaryl,
(i) $-SO_2NHCOR^{2'}$,
(j) $-CH_2SO_2NHCOR^{2'}$,
(k) $-CONHSO_2R^{2'}$,
(l) $-CH_2CONHSO_2R^{2'}$,
(m) $-NHSO_2NHCOR^{2'}$,
(n) $-NHCONHSO_2R^{2'}$,
(o) $-SO_2NHCONHR^{2'}$,
(p) $-SO_2NHCN$,
(q) $-CONHOR^5$,

(r) 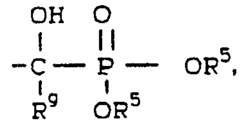

(s) 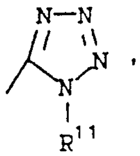

(t) 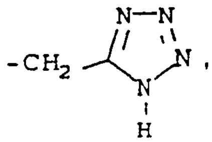

(u) 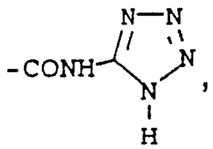

(v) $-CONHNHSO_2CF_3$ ,

(w) 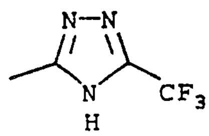

(x) 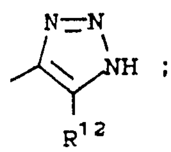

(y) 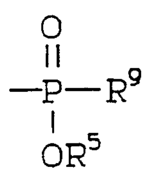

wherein:

heteroaryl is an unsubstituted, monosubstituted or disubstituted 5- or 6-membered aromiatic ring which can optionally contain from 1 to 3 heteroatoms selected from the group consisting of O, N and S and wherein the substituents are members selected from the group consisting of -OH, -SH, $C_1$-$C_4$-alkyl, $C_1$-$C_4$-alkoxy, $-CF_3$, halo(Cl, Br, F, I), $-NO_2$, $-CO_2H$, $-CO_2$-$C_1$-$C_4$-alkyl. $-NH_2$, -NH($C_1$-$C_4$-alkyl) or -N($C_1$-$C_4$-alkyl)$_2$;

$R^{2a}$ and $R^{2b}$ are independently H, halo (Cl, Br, I, F), $-NO_2$, $-NH_2$, $C_1$-$C_4$-alkylamino, $-SO_2NHR^9$, $CF_3$ $C_1$-$C_4$-alkyl, or $C_1$-$C_4$-alkoxy;

$R^{3a}$ is

(a) H,

(b) halo(Cl, Br, I, F)

(c) $C_1$-$C_6$-alkyl,

(d) $C_1$-$C_6$-alkoxy,
(e) $C_1$-$C_6$-alkoxyalkyl;
$R^{3b}$ is
(a) H,
(b) halo (Cl, Br, I, F)
(c) $NO_2$,
(d) $C_1$-$C_6$-alkyl,
(e) $C_1$-$C_6$-acyloxy,
(f) $C_1$-$C_6$-cycloalkyl
(g) $C_1$-$C_6$-alkoxy,
(h) -$NHSO_2R^4$,
(i) hydroxy $C_1$-$C_4$-alkyl,
(j) aryl $C_1$-$C_4$-alkyl
(k) $C_1$-$C_4$-alkylthio
(l) $C_1$-$C_4$-alkyl sulfinyl
(m) $C_1$-$C_4$-alkyl sulfonyl
(n) $NH_2$
(o) $C_1$-$C_4$-alkylamino
(p) $C_1$-$C_4$-dialkylamino
(q) fluoro $C_1$-$C_4$-alkyl
(r) -$SO_2$-$NHR^9$
(s) aryl or,
(t) furyl;
wherein aryl is phenyl or naphthyl optionally substituted with one or two substituents selected from the group consisting of halo(Cl, Br, I, F) $C_1$-$C_4$-alkyl, $C_1$-$C_4$-alkoxy, $NO_2$, $CF_3$, $C_1$-$C_4$-alkylthio, OH or $NH_2$;
$R^4$ is H, straight chain or branched $C_1$-$C_6$ alkyl, aryl or -$CH_2$-aryl where aryl is as defined above;
$R^{4a}$ is $C_1$-$C_6$-alkyl, aryl, or -$CH_2$-aryl where aryl is as defined above;

$R^5$ is H, 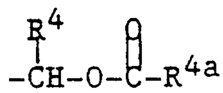;

E is a single bond, -$NR^{13}(CH_2)_s$-,-$S(O)_x(CH_2)_s$-where x is 0 to 2 and s is 0 to 5, -CH(OH)-, -O-, -CO-;
$R^6$ is
(a) aryl as defined above optionally substituted with 1 or 2 substituents selected from the group consisting of halo (Cl, Br, I, F) -O-$C_1$-$C_4$-alkyl, $C_1$-$C_4$-alkyl, -$NO_2$, -$CF_3$, -$SO_2NR^9R^{10}$, -S-$C_1$-$C_4$-alkyl, -OH, -$NH_2$, $C_3$-$C_7$-cycloalkyl, $C_3$-$C_{10}$-alkenyl;
(b) straight chain or branched $C_1$-$C_6$-alkyl, $C_1$-$C_5$-alkenyl or $C_1$-$C_5$-alkynyl each of which can be optionally substituted with a substituent selected from the group consisting of aryl as defined above, $C_3$-$C_7$-cycloalkyl, halo (Cl, Br, I, F) -OH, -$NH_2$, -NH ($C_1$-$C_4$-alkyl), -N($C_1$-$C_4$-alkyl$)_2$, -NH-$SO_2R^4$, -$COOR^4$, -$SO_2NHR^9$; or
(c) an unsubstituted, monosubstituted or disubstituted aromatic 5 or 6 membered cyclic ring which can contain one or two members selected from the group consisting of N, O, S, and wherein the substituents are members selected from the group consisting of -OH, -SH, $C_1$-$C_4$-alkyl, $C_1$-$C_4$-alkyloxy -$CF_3$, halo (Cl, Br, I, F), or $NO_2$;
$R^{7a}$ and $R^{7b}$ are independently
(a) H,
(b) straight chain or branched $C_1$-$C_6$-alkyl, $C_1$-$C_6$-alkenyl or $C_1$-$C_6$-alkynyl,
(c) halo(Cl, Br, I, F)
(d) perfluoro-$C_1$-$C_4$-alkyl,
(e) $C_3$-$C_5$-cycloalkyl, or
(f) when $R^{7a}$ and $R^{7b}$ are bonded to adjacent carbon atoms, they can be joined to form an aryl or heteroaryl ring wherein the aryl is as defined above and the heteroaryl is as defined below;
$R^{8a}$ and $R^{8b}$ are independently
(a) H,
(b) aryl-$C_1$-$C_4$-alkyl,
(c) heteroaryl-$C_1$-$C_4$-alkyl,
(d) $C_1$-$C_4$-alkyl, $C_2$-$C_6$-alkenyl or $C_2$-$C_6$-alkynyl each of which can be optionally substituted with a

substituent selected from the group consisting of -OH, -SH, $-NH_2$, amidino, guanidino, cyano, $C_1$-$C_4$-alkoxy, $C_1$-$C_4$-alkylthio, ($C_1$-$C_4$-alkyl)amino, di-($C_1$-$C_4$-alkyl)amino, $-COOR^4$, $-CON(R^4)_2$, $-O-COR^4$, $-N(R^4)-COR^4$, $-CON(CH_2-CH_2)_2Z$, -NHCOO($C_1$-$C_4$-alkyl), $S(O)_xR^{21}$, $-CONHSO_2R^{21}$, $-SO_2NHCOR^{21}$, $-SO_2NH$-heteroaryl as defined below, tetrazol-5-yl, $-PO(OR^4)_2$, $-PO(OR^4)R^9$,or heteroaryl as defined below;

(e) -CO-aryl,
(f) $C_3$-$C_7$-cycloalkyl,
(g) halo (Cl, Br, I, F),
(h) -OH,
(i) $-OR^{21}$,
(j) -perfluoro-$C_1$-$C_4$-alkyl,
(k) -SH,
(l) $-S(O)_x-R^{21}$ where x is as defined above,
(m) -CHO,
(n) $-CO_2H$,
(o) $-CO_2-C_1-C_4$-alkyl,
(p) $-SO_3H$,
(q) $-NH_2$,
(r) -NH($C_1$-$C_4$-alkyl),
(s) -N($C_1$-$C_4$-alkyl$)_2$,
(t) $-NHCO_2-C_1-C_4$-alkyl, (u) $-SO_2NHR^9$,
(v) $-SO_2NR^9R^{10}$,
(w) $-NO_2$,
(x) $-NH-SO_2-R^{21}$,
(y) aryl,
(z) heteroaryl,
(aa) -NH-($C_3$-$C_7$-cycloalkyl), (bb)

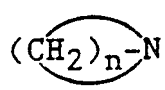

where n is 4 to 6,
(cc) -CN,
(dd) tetrazol-5-yl,
(ee) $-CONHSO_2R^{21}$,
(ff) $-SO_2NHCOR^{21}$,
(gg) $-SO_2NH$-heteroaryl,
(hh) $-N(R^4)-CON(R^4)-R^{21}$,
(ii) $-PO(OR^4)_2$,
(jj) $-PO(OR^4)R^9$,
(kk) $-CON(CH_2-CH_2)_2$ Z, where Z is as defined below,
(ll) $-N(R^4)-COOR^{21}$,
(mm)

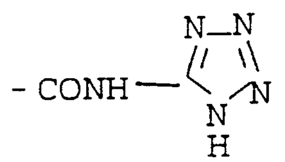 ,

(nn) $-N(CH_2-CH_2)_2-Z$ where Z is as defined below,
(oo) -NHCO-heteroaryl,
wherein aryl is defined above and heteroaryl is a 5 or 6 membered heterocyclic ring containing up to three heteroatoms selected from the group consisting of O, N, or S wherein S may be in the form of sulfoxide or sulfone and wherein the heteroaryl may be substituted with halo (F, Cl, Br, I), $C_1$-$C_4$-alkyl, $C_1$-$C_4$-alkoxy, $NO_2$, OH, COOH, COO($C_1$-$C_4$-alkyl), $N(R^4)_2$,
$R^9$ is H, $C_1$-$C_5$-alkyl, aryl, or $-CH_2$-aryl;

$R^{10}$ is H, $C_1$-$C_4$-alkyl;
$R^{11}$ is H, $C_1$-$C_6$-alkyl, $C_2$-$C_4$-alkenyl, $C_1$-$C_4$-alkoxy or $-CH_2-C_6H_4R^{20}$;
$R^{12}$ is -CN, $-NO_2$ or $-CO_2R^4$;
$R^{13}$ is H, -CO($C_1$-$C_4$-alkyl), $C_1$-$C_6$-alkyl, allyl, $C_3$-$C_6$-cycloalkyl, phenyl or benzyl;
$R^{14}$ is H, $C_1$-$C_8$-alkyl, $C_1$-$C_8$-perfluoroalkyl, $C_3$-$C_6$-cycloalkyl, phenyl or benzyl;
$R^{15}$ is H, $C_1$-$C_6$-alkyl;
$R^{16}$ is H, $C_1$-$C_6$-alkyl, $C_3$-$C_6$-cycloalkyl, phenyl or benzyl;
$R^{17}$ is $-NR^9R^{10}$, $-OR^{10}$, $-NHCONH_2$, $-NHCSNH_2$,

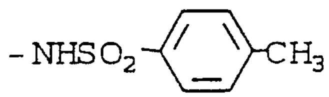 or 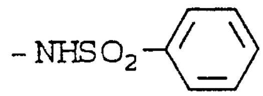 ;

$R^{18}$ and $R^{19}$ are independently $C_1$-$C_4$-alkyl or taken together are $-(CH_2)_q$-where q is 2 or 3;
$R^{20}$ is H, $-NO_2$, $-NH_2$, -OH or $-OCH_3$;
$R^{21}$ is
(a) aryl as defined above,
(b) heteroaryl as first defined above,
(c) $C_3$-$C_7$-cycloalkyl,
(d) perfluoro-$C_1$-$C_4$-alkyl,
(e) $C_1$-$C_4$-alkyl optionally substituted with a substituent selected from the group consisting of aryl as defined above, heteroaryl as defined above, -OH, -SH, $C_1$-$C_4$-alkyl, -O($C_1$-$C_4$-alkyl), S($C_1$-$C_4$-alkyl), $-CF_3$, halo(Cl, Br, F, I), $-NO_2$, $-CO_2H$, $CO_2$-$C_1$-$C_4$-alkyl, $-NH_2$, -NH($C_1$-$C_4$-alkyl), -N($C_1$-$C_4$-alkyl)$_2$, $-PO_3H$, -PO(OH)(O-$C_1$-$C_4$-alkyl);
X is absent or is
(a) a carbon-carbon single bond,
(b) -CO-,
(c) -O-,
(d) -S-,

(e) 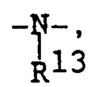

(f) 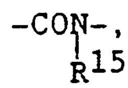

(g) 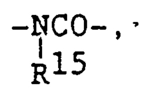

(h) $-OCH_2-$,
(i) $-CH_2O-$
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,
(p) -CH=CH-,
(q) -CF=CF-,
(r) -CH=CF-,
(s) -CF=CH-,
(t) $-CH_2CH_2-$,
(u) $-CF_2CF_2-$,
(v) 1,1 and 1,2-disubstituted cyclopropyl,

(w) 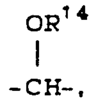

(x) 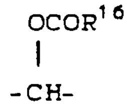

(y) 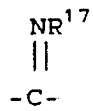 , or

(z) 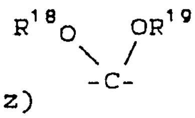 ;

is O, $NR^{13}$ or S;
r is 1 or 2; and,
the pharmaceutically acceptable salts thereof.

2. A pharmaceutical composition useful in the treatment of hypertension which comprises a pharmaceutically acceptable carrier and a pharmaceutically effective amount of a compound of Claim 1:

3. The composition of Claim 2 which includes an antihypertensive or a diuretic or an angiotensin converting enzyme or a calcium channel blocker which are members selected from the group consisting of: amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methyclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate, benzthiazide, quinethazone, ticrynafan, triamterene, acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, as well as admixtures and combinations thereof.

4. An ophthalmological formulation for the treatment of ocular hypertension comprising an ophthalmologically acceptable carrier and an effective ocular antihypertensive amount of a compound of Claim 1.

European Patent Office

**PARTIAL EUROPEAN SEARCH REPORT**
which under Rule 45 of the European Patent Convention shall be considered, for the purposes of subsequent proceedings, as the European search report

Application number
EP 90 30 5179

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A- 0 186 190 (SUMITOMO CHEMICAL CO. LTD)<br>-- | | C 07 D 235/08<br>A 61 K 31/415<br>C 07 D 235/02<br>C 07 D 403/10<br>C 07 D 403/12<br>A 61 K 31/505<br>A 61 K 31/41<br>C 07 F 9/6506 |
| A | EP-A-0 132 606 (SUMITOMO CHEMICAL CO. LTD)<br>-- | | |
| D,A | EP-A-0 253 310 (E.I.DU PONT DE NEMOURS AND CO.)<br>-- | | |
| D,A | EP-A-0 291 969 (E.I. DU PONT DE NEMOURS AND CO.)<br>-- | | |
| D,A | EP-A-0 028 834 (TAKEDA YAKUHIN KOGYO K.K.)<br>--<br>./. | | TECHNICAL FIELDS SEARCHED (Int. Cl.4)<br>C 07 D 235/00<br>A 61 K 31/00<br>C 07 D 403/00<br>C 07 F 9/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.
Claims searched completely:
Claims searched incompletely:
Claims not searched:
Reason for the limitation of the search:

As the draft of the claims is not clear and concise (Art. 83-84 EPO) and encompasses such an enormous amount of products, a complete search is not possible on economic grounds (see Guidelines for Examination in the EPO, Part B, Chapter III, 2.) So the search has been limited (Rule 45) to the examples.

| Place of search | Date of completion of the search | Examiner |
|---|---|---|
| THE HAGUE | 22-08-1990 | DE BUYSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

**PARTIAL EUROPEAN SEARCH REPORT**

Application number EP 90 30 5179

-2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| P,D, X | US-A-4 880 804 (DAVID J. CARINI, A.O.(E.I. DU PONT DE NEMOURS AND CO.) ) | | |
| | * The whole document * | 1-2 | |
| | -- | | |
| P,A | EP-A-0 360 098 (SUMITOMO CHEMICAL CO. LTD) | | |
| | ---- | | TECHNICAL FIELDS SEARCHED (Int Cl 4) |